(19) Europäisches Patentamt
European Patent Office
Office européen des brevets

(11) EP 3 281 623 B1

(12) **EUROPEAN PATENT SPECIFICATION**

(45) Date of publication and mention of the grant of the patent:
**31.07.2019 Bulletin 2019/31**

(51) Int Cl.:
***A61K 8/898*** *(2006.01)* ***A61Q 5/06*** *(2006.01)*
***A61Q 5/10*** *(2006.01)*

(21) Application number: **16184012.9**

(22) Date of filing: **12.08.2016**

(54) **HAIR COLORING COMPOSITION FOR PROVIDING A FILM ON KERATIN FIBERS**

HAARFÄRBEZUSAMMENSETZUNG ZUM AUFBRINGEN EINES FILMS AUF KERATINFASERN

COMPOSITION DE COLORATION CAPILLAIRE PERMETTANT DE DÉPOSER UN FILM SUR DES FIBRES DE KÉRATINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**14.02.2018 Bulletin 2018/07**

(73) Proprietor: **Noxell Corporation**
**Hunt Valley, Maryland 21030-2098 (US)**

(72) Inventors:
- **Herrlein, Mathias Kurt**
  **65824 Schwalbach am Taunus (DE)**
- **Forgione, Marianna**
  **65824 Schwalbach am Taunus (DE)**
- **Schäfer, Tatjana**
  **65824 Schwalbach am Taunus (DE)**
- **Heppenheimer, Kurt**
  **65824 Schwalbach am Taunus (DE)**

(74) Representative: **Hanna Moore + Curley**
**Garryard House**
**25/26 Earlsfort Terrace**
**Dublin 2, D02 PX51 (IE)**

(56) References cited:
**WO-A1-2009/061360 US-A1- 2009 074 701**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 281 623 B1

## Description

### FIELD OF THE INVENTION

The present invention relates to the field of cosmetics, particularly hair cosmetics, and relates to hair coloring compositions for providing a film on keratin fibers. It also relates to the use of the hair coloring composition, a method for providing a film on keratin fibers and a kit.

### BACKGROUND OF THE INVENTION

Semi-permanent treatments to human keratin fibers are well known in the art. Of particular note are semi-permanent treatments that alter the color appearance of the hair or provide other colored or reflective properties via the use of glitter or particles. For example, direct dyes color the hair in a semi-permanent fashion by adhering colored molecules to the keratin fibers. The dye can be later washed out. Hair chalks are powder-based or powdery products - typically provided in a blusher-style 'compact' or in pen format - that enable the user to apply pigments and/or colored particles to the hair.

A drawback of the known technology in this area is low adherence of the pigment or colored material to the keratin fibers - it is a significant consumer concern that such products can make your clothes and/or bathroom dirty and/or stained. Furthermore, such pigment or colored material can migrate to the skin on your neck, shoulders and face and cause unsightly marks. Moreover, consumers wish to be able to apply. Chalk products typically provide a matt look, which may not be desired by all consumers and would not provide a vibrant, shiny look where the consumer desires this.

Thus there is a need for compositions and methods that provide more durable means to adhere pigments and/or colored/shiny material to keratin fibers, but also any type of dye composition such a dye composition comprising one or more direct dyes or containing oxidation dye precursors. Oxidation dye precursors, or oxidation bases, are colorless or slightly colored compounds which, when mixed with oxidizing products at the time of use, are able, by a process of oxidative condensation, to give rise to colored compounds and dyes. It is also known that it is possible to vary the shades obtained with these oxidation bases by combining them with couplers or dye modifiers. Hence, there is a need for providing improved deposition of all these different dyeing ingredients.

In addition, there is a need for such a composition that can be easily applied and distributed over the hair - for example does not result in clumping of the composition, or any kind of gluey-ness or gunky-ness of any kind on the head of hair. Indeed, there is a need for a more natural look to be provided by such means.

### SUMMARY OF THE INVENTION

A hair coloring composition for providing a film on keratin fibers is provided and comprises in a cosmetically acceptable carrier:

(a) a film-forming aminosilicone polymer, wherein the film-forming aminosilicone polymer is the product of the reaction of:

a1) a siloxane comprising at least two oxirane or oxetane groups having the formula:

$$M_fM^E_hM^{PE}_iM^H_jD_kD^E_lD^{PE}_mD^H_nT_oT^E_pT^{PE}_qT^H_rQ_s$$

With

$M = R^9R^{10}R^{11}SiO_{1/2}$;
$M^H = R^{12}R^{13}HSiO_{1/2}$;
$M^{PE}=R^{12}R^{13}(-CH_2CH(R^{14})(R^{15})_tO(R^{16})_u(C_2H_4O)_v(C_3H_6O)_w(C_4HsO)_xR^{17})SiO_{1/2}$;
$M^E = R^{12}R^{13}(R^E)SiO_{1/2}$;
$D= R^{18}R^{19}SiO_{2/2}$; and
$D^H = R^{20}HSiO_{2/2}$;
$D^{PE} = R^{20}(-CH_2CH(R^{14})(R^{15})_tO(R^{16})_u(C_2H_4O)_v(C_3H_6O)_w(C_4H_8O)_xR^{17})SiO_{2/2}$;
$D^E = R^{20}R^ESiO_{2/2}$;
$T = R^{21}SiO_{3/2}$;
$T^H = HSiO_{3/2}$;
$T^{PE} = (-CH_2CH(R^{14})(R^{15})_tO(R^{16})_u(C_2H_4O)_v(C_3H_5O)_w(C_4H_8O)_xR^{17})SiO_{3/2}$;
$T^E = R^ESiO_{3/2}$; and

$Q = SiO_{4/2}$;

Where

$R^9$, $R^{10}$, $R^{11}$, $R^{12}$, $R^{13}$, $R^{18}$, $R^{19}$, $R^{20}$, and $R^{21}$ are each independently selected from the group of monovalent hydrocarbon radicals having from 1 to 60 carbon atoms;

$R^{14}$ is H or a 1 to 6 carbon atom alkyl group;

$R^{15}$ is a divalent alkyl radical of 1 to 6 carbons;

$R^{16}$ is selected from the group of divalent radicals consisting of $-C_2H_4O-$, $-C_3H_6O-$, and $-C_4H_8O-$;

$R^{17}$ is selected from the group consisting of H, monofunctional hydrocarbon radicals of 1 to 6 carbons, and acetyl;

$R^E$ is independently a monovalent hydrocarbon radical containing one or more oxirane or oxetane moieties having from 1 to 60 carbon atoms;

the subscript f is 0 or positive subject to the limitation that when the subscript f is 0, h must be positive;

the subscript h is 0 or positive subject to the limitations that when h is 0, the subscript f is positive, and that the sum of the subscripts h, 1 and p is positive;

the subscript k is zero or positive and has a value ranging from 0 to 1000;

the subscript 1 is 0 or positive and has a value ranging from 0 to 400 subject to the limitation that the sum of the subscripts h, 1 and p is positive;

the subscript o is 0 or positive and has a value ranging from 0 to 50;

the subscript p is 0 or positive and has a value ranging from 0 to 30 subject to the limitation that the sum of the subscripts h, 1 and p is positive;

the subscript s is 0 or positive and has a value ranging from 0 to 20;

the subscript i is 0 or positive and has a value ranging from 0 to 20;

the subscript m is 0 or positive and has a value ranging from 0 to 200;

the subscript q is 0 or positive and has a value ranging from 0 to 30;

the subscript j is 0 or positive and has a value ranging from 0 to 2;

the subscript n is 0 or positive and has a value ranging from 0 to 20;

the subscript r is 0 or positive and has a value ranging from 0 to 30;

the subscript t is 0 or 1;

the subscript u is 0 or 1;

the subscript v is 0 or positive and has a value ranging from 0 to 100 subject to the limitation that $(v+w+x) > 0$;

the subscript w is 0 or positive and has a value ranging from 0 to 100 subject to the limitation that $(v+w+x) > 0$;

the subscript x is 0 or positive and has a value ranging from 0 to 100 subject to the limitation that $(v + w + x) > 0$; and

a2) an aminosilane having the formula:

$$N(H)(R^1)R^2Si(OR^3)_{3-ab-c}(OR^4)_a(R^5Si(OR^6)_d(R^7)_e)_bR^8_c$$

with

$R^1$ is chosen from the group consisting of H or a monovalent hydrocarbon radical containing 1 to 20 carbon atoms;

$R^2$ is selected from a group consisting of a divalent linear or branched hydrocarbon radical consisting of 1 to 60 carbons;

$R^4$ is a hydrocarbon radical that contains 3 to 200 carbon atoms;

$R^5$ is selected from a group consisting of oxygen or a divalent linear or branched hydrocarbon radical consisting of 1 to 60 carbons;

$R^3$, $R^6$, $R^7$, and $R^8$ and are each independently selected from the group of monovalent linear or branched hydrocarbon radicals having from 1 to 200 carbon atoms;

the subscript b is 0 or a positive number and has a value ranging from 0 to 3;

the subscript a is a positive number less than 3;

the subscripts b and c are 0 or positive and have a value ranging from 0 to 3 subject to the limitation that $(a+b+c) \leq 3$;

the subscripts d and e are 0 or positive and have a value ranging from 0 to 3 subject to the limitation that $(d+e) = 3$; and

(b) a hair coloring agent.

Use of the hair coloring composition as stated hereinbefore for highlighting already dyed hair fibers.

A method for providing a film onto keratin fibers is provided and comprises applying the hair coloring composition as stated hereinbefore onto keratin fibers and allowing the keratin fibers to dry or drying them.

A kit is provided and comprises:

◦ the hair coloring composition as stated hereinbefore; and
◦ an applicator.

The hair coloring agent may comprise one or more pigments wherein the one or more pigments are selected from the group consisting of metal oxides, hydroxides and oxide hydrates, mixed phase pigments, sulfur-containing silicates, metal sulfides, complex metal cyanides, metal sulfates, chromates and molybdates, and the metals themselves, and combinations thereof, preferably wherein the pigments are selected from the group consisting of iron oxide, titanium dioxide, mica, borosilicate, and combinations thereof.

The hair coloring composition may further comprise a thickening system comprising:

a deposition enhancer, wherein the deposition enhancer is a hydrophilic and non-ionic polymer, and wherein the deposition enhancer has a weight average molecular weight of from 700,000 Dalton to 3,000,000 Dalton; and
a thickening polymer, wherein the thickening polymer has a weight average molecular weight of at least 10,000 Dalton, and wherein the thickening polymer is a cationic thickening polymer or is a non-ionic thickening polymer. The thickening polymer may be a hydroxyethyl cellulose.

The deposition enhancer may conform to the formula $H(OCH_2CH_2)_nOH$ wherein n has an average value of from 20,000 to 50,000.

The hair coloring composition may comprise from 1% to 20% of the film-forming aminosilicone polymer (a), by total weight of the composition.

DETAILED DESCRIPTION OF THE INVENTION

Definitions and general

In this document, including in all embodiments of all aspects of the present invention, the following definitions apply unless specifically stated otherwise.

All percentages are by weight (w/w) of the total composition, unless otherwise specified. All ratios are weight ratios, unless otherwise specified. "wt%" means percentage by weight. References to 'parts' e.g. a mixture of 1 part X and 3 parts Y, is a ratio by weight.

"QSP" means sufficient quantity for 100% or for 100g. +/- indicates the standard deviation. All ranges are inclusive and combinable. The number of significant digits conveys neither a limitation on the indicated amounts nor on the accuracy of the measurements. All numerical amounts are understood to be modified by the word "about".

All measurements are understood to be made at 20°C and at ambient conditions, where "ambient conditions" means at 1 atmosphere (atm) of pressure and at 65% relative humidity. "Relative humidity" refers to the ratio (stated as a percent) of the moisture content of air compared to the saturated moisture level at the same temperature and pressure. Relative humidity can be measured with a hygrometer, in particular with a probe hygrometer from VWR® International.

Herein "min" means "minute" or "minutes". Herein "mol" means mole. Herein "g" following a number means "gram" or "grams". All amounts as they pertain to listed ingredients are based on the active level ('solids') and do not include carriers or by-products that may be included in commercially available materials.

Herein, "comprising" means that other steps and other ingredients can be in addition. "Comprising" encompasses the terms "consisting of' and "consisting essentially of'. The hair coloring compositions, methods, uses, and kits of the present invention can comprise, consist of, and consist essentially of the elements and limitations of the invention described herein, as well as any of the additional or optional ingredients, components, steps, or limitations described herein. Embodiments and aspects described herein may comprise or be combinable with elements, features or components of other embodiments and/or aspects despite not being expressly exemplified in combination, unless an incompatibility is stated.

Where amount ranges are given, these are to be understood as being the total amount of said ingredient in the composition, or where more than one species fall within the scope of the ingredient definition, the total amount of all ingredients fitting that definition, in the composition. For example, if the composition comprises from 1% to 5% fatty alcohol, then a composition comprising 2% stearyl alcohol and 1% cetyl alcohol and no other fatty alcohol, would fall within this scope.

The amount of each particular ingredient or mixtures thereof described hereinafter can account for up to 100%

(or 100%) of the total amount of the ingredient(s) in the composition.

The term "substantially free of' as used herein means less than 1%, less than 0.8%, less than 0.5%, less than 0.3%, or less than an immaterial amount of by total weight of the composition.

"Viscosity" is measured at 23°C using a HAAKE Rotation Viscometer VT 550 with cooling/heating vessel and sensor systems according to DIN 53019 at a shear rate of 12.9 $s^{-1}$.

The term "molecular weight" or "M.Wt." as used herein refers to the weight average molecular weight unless otherwise stated. The weight average molecular weight may be measured by gel permeation chromatography.

The term "Polymer" as used herein means a chemical formed from the polymerisation of two or more monomers. The term "polymer" shall include all materials made by the polymerisation of monomers as well as natural polymers. Polymers made from only one type of monomer are called homopolymers. Herein, a polymer comprises at least two monomers. Polymers made from two or more different types of monomers are called copolymers. The distribution of the different monomers can be random, alternating or block-wise (i.e. block copolymer). The term "polymer" used herein includes any type of polymer including homopolymers and copolymers.

The term "hair" as used herein means mammalian hair including scalp hair, facial hair and body hair, more preferably hair on the human head and scalp. Hair comprises hair fibers. "Hair shaft" means an individual hair strand and may be used interchangeably with the term "hair." As used herein the term "hair" to be treated may be "living" i.e. on a living body or may be "non-living" i.e. in a wig, hairpiece or other aggregation of non-living keratinous fibers. Mammalian, preferably human hair is preferred.

"Proximal to the scalp" means that portion of an extended, or substantially straightened, hair shaft that is closer in distance to the scalp than to the end of the hair. Thus, 50% of the hair fiber length would be considered proximal to the scalp, and 50% of the hair fiber would be distal to the scalp. "z cm proximal to the scalp" means a distance "z" along the hair, with one endpoint being on or directly adjacent to the scalp, and the second endpoint being measured "z" centimetres along the length of the extended or substantially straightened hair.

The term "film-forming aminosilicone polymer" as used herein means an aminosilicone polymer which is in a position to deposit a polymer film on the hair.

The term "cosmetically acceptable" as used herein means that the compositions, or components described are suitable for use in contact with human keratinous tissue without undue toxicity, incompatibility, instability, allergic response, and the like. All compositions described herein which have the purpose of being directly applied to keratinous tissue are limited to those being cosmetically acceptable.

The term "separately packaged" as used herein means any form of packaging that prevents a first composition from coming into physical contact, or admixing, with a second composition. "Separately packaged" may mean that the individual first, and second compositions are packaged in separate containers, or alternatively in a single container partitioned such that the first and second compositions are not in physical contact.

The term "kit" as used herein means a packaging unit comprising a plurality of components i.e. a kit of parts. An example of a kit is, for example, a first composition, and a separately packaged second composition. Another kit may comprise application instructions comprising a method and a first and second compositions.

A hair coloring composition for providing a film on keratin fibers is provided. Indeed, the combination of a film-forming aminosilicone polymer as set out more in detail hereinafter, and a hair coloring agent in a cosmetically acceptable carrier results in a film on keratin fibers, particularly human head hair, that is sufficiently durable that it does not quickly rub off whilst on the keratin fibers, for example on the hands, clothes etc of the consumer, nor does it provide bad hair feel, such as lumpiness, nor abrasiveness, nor weigh down the hair reducing the head of hair volume, nor unsightly globules.

Indeed, the film-forming aminosilicone polymer enables the forming of a very thin, dry film, which tightly binds any hair coloring agent, e.g. pigment and/or colored material and/or direct dyes and/or oxidation dyes onto the hair fibers in a durable fashion. Hence, the film-forming aminosilicone polymer provides an excellent film.

The provision of the film-forming aminosilicone polymer in the hair coloring composition can help to provide an excellent support system for any type of hair coloring agent such as pigment and/or colored material and/or direct dyes and/or oxidation dyes, which can provide the desired effects to the hair, such as glitteriness, color, shininess etc.

## Hair coloring composition

The hair coloring composition as set out herein above is for providing a film on keratin fibers. The hair coloring composition may be for providing a film, preferably comprising pigment or colored material on keratin fibers. The film may be a durable film.

The hair coloring composition may be substantially free of compounds causing precipitation of any component of the hair coloring composition when the hair coloring composition is in aqueous solution at pH 5 and at 23°C.

Indeed, precipitation of any component of the present composition would have negative side effects on the efficacy of the present invention - particularly in terms of hair feel. Indeed, with the exclusion of the hair coloring agent

that is herein intended to be immobilised on hair, other residues on hair are not accepted by the consumer. For example, precipitation of the film-forming aminosilicone polymer would detract from their ability to form a film on the hair fibers and would form residues on hair. The hair coloring composition may be substantially free of any further ionic compounds. The hair coloring composition may be substantially free of any compounds that are cationic at pH 4.0 to 5.0.

Cosmetically acceptable carrier

The hair coloring composition comprises a cosmetically acceptable carrier. The cosmetically acceptable carrier of the hair coloring composition may be an aqueous carrier. The hair coloring composition may comprise water. Water can provide a hydrophilic phase, which the hydrophilic portions of any other ingredients comprised in the hair coloring composition can interact with water. Water can also provide a fluid phase meaning that the hair coloring composition can be in liquid form. The hair coloring composition may comprise from 50% to 85% water, or from 65% to 75% of water by total weight of the composition.

The cosmetically acceptable carrier is any carrier suitable for formulating the hair coloring composition being suitable for application onto hair. The cosmetically acceptable carrier may be selected from either an aqueous medium or an aqueous-alcoholic medium. When the cosmetically acceptable carrier is an aqueous-alcoholic carrier, the cosmetically acceptable carrier may comprise water and an alcohol. An alcohol can advantageously influence the viscosity of a relatively wide spectrum of ingredients of the composition. The alcohol of the hair coloring composition may be selected from the group consisting of ethanol, isopropanol, propanol, and mixtures thereof.

When the cosmetically acceptable carrier is an aqueous carrier, the aqueous carrier may consist essentially of water and may be substantially free of alcohol. The hair coloring composition may comprise a safe and effective amount of a cosmetically acceptable carrier which is water. The hair coloring composition may comprise from 0.1% to 99%, or from 1% to 98%, or from 10% to 97%, or from 30% to 95% of water by total weight of the composition.

When used daily, organic solvents in the hair coloring compositions may have a negative effect on humans and on the environment. They also have an unpleasant odor. Hence, the hair coloring composition may be substantially free of organic solvents. Organic solvents may be any volatile alcohols, e.g. ethanol, isopropanol, propanol.

The hair coloring composition may be substantially free of alcohol, such as volatile alcohols (e.g. ethanol, isopropanol, propanol). When the hair coloring composition is substantially free of alcohol, the a hair coloring composition can advantageously have a reduced odour. Flammability issues can also be prevented.

The cosmetically acceptable carrier of the hair coloring composition may be an oily compound. The oily compound may be selected from the group consisting of cyclic silicones and volatile hydrocarbons. Cyclic silicones can be available from Dow Corning. The cyclic silicone may have from at least 3 silicone atoms or from at least 5 silicone atoms but no more than 7 silicone atoms or no more than 6 silicone atoms. The cyclic silicone may conform to the formula:

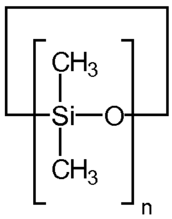

wherein n is from 3 or from 5 but no more than 7 or no more than 6. The cyclic silicone may have a kinematic viscosity of less than 10 cPs at 23°C. A Suitable cyclic silicone for use herein may include Cyclomethicone D5 (commercially available from G.E. Silicones). Alternatively, the hair coloring composition may be silicone-free.

Volatile hydrocarbons e.g. Isopar can be obtained from ExxonMobil Petroleum and Chemical. The oily compound may be a mineral oil. Trade names for suitable mineral oils include Benol, Blandol, Hydrobrite, Kaydol (Sonneborn LLC Refined Products), Chevron Superla White Oil (Chevron Products Company), Drakeol, Parol (Calumet Penreco LLC), Peneteck (Calumet Penreco LLC), Marcol, and Primol 352 (ExxonMobil Petroleum and Chemical).

Film-forming aminosilicone polymer

The hair coloring composition comprises a film-forming aminosilicone polymer. The film-forming aminosilicone polymer of the present invention is described in European Patent EP 2 214 633 B1 in par. 11-12.

The film-forming aminosilicone polymer is the product of the reaction of a siloxane and an aminosilane.

The siloxane comprises at least two oxirane or oxetane groups having the formula:

$$M_f M^E_h M^{PE}_i M^H_j D_k D^E_l D^{PE}_m D^H_n T_o T^E_p T^{PE}_q T^H_r Q_s$$

with

$M = R^9R^{10}R^{11}SiO_{1/2}$;
$M^H = R^{12}R^{13}HSiO_{1/2}$;
$M^{PE}=R^{12}R^{13}(-CH_2CH(R^{14})(R^{15})_tO(R^{16})_u(C_2H_4O)_v(C_3H_6O)_w(C_4HsO)_xR^{17})SiO_{1/2}$;
$M^E = R^{12}R^{13}(R^E)SiO_{1/2}$;
$D= R^{18}R^{19}SiO_{2/2}$; and
$D^H = R^{20}HSiO_{2/2}$;
$D^{PE} = R^{20}(-CH_2CH(R^{14})(R^{15})_tO(R^{16})_u(C_2H_4O)_v(C_3H_6O)_w(C_4H_8O)_xR^{17})SiO_{2/2}$;
$D^E = R^{20}R^ESiO_{2/2}$;
$T = R^{21}SiO_{3/2}$;
$T^H = HSiO_{3/2}$;
$T^{PE} = (-CH_2CH(R^{14})(R^{15})_tO(R^{16}(C_2H_4O)_v(C_3H_5O)_w(C_4H_8O)_xR^{17})SiO_{3/2}$;
$T^E = R^ESiO_{3/2}$; and
$Q = SiO_{4/2}$; where
$R^9$, $R^{10}$, $R^{11}$, $R^{12}$, $R^{13}$, $R^{18}$, $R^{19}$, $R^{20}$, and $R^{21}$ are each independently selected from the group of monovalent hydrocarbon radicals having from 1 to 60 carbon atoms;
$R^{14}$ is H or a 1 to 6 carbon atom alkyl group; $R^{15}$ is a divalent alkyl radical of 1 to 6 carbons; $R^{16}$ is selected from the group of divalent radicals consisting of $-C_2H_4O-$, $-C_3H_6O-$, and $-C_4H_8O-$; $R^{17}$ is selected from the group consisting of H, monofunctional hydrocarbon radicals of 1 to 6 carbons, and acetyl;
$R^E$ is independently a monovalent hydrocarbon radical containing one or more oxirane or oxetane moieties having from 1 to 60 carbon atoms;
the subscript f is 0 or positive subject to the limitation that when the subscript f is 0, h must be positive;
the subscript h is 0 or positive subject to the limitations that when h is 0, the subscript f is positive, and that the sum of the subscripts h, 1 and p is positive;
the subscript k is zero or positive and has a value ranging from 0 to 1000;
the subscript 1 is 0 or positive and has a value ranging from 0 to 400 subject to the limitation that the sum of the subscripts h, 1 and p is positive;
the subscript o is 0 or positive and has a value ranging from 0 to 50;
the subscript p is 0 or positive and has a value ranging from 0 to 30 subject to the limitation that the sum of the subscripts h, 1 and p is positive;
the subscript s is 0 or positive and has a value ranging from 0 to 20;
the subscript i is 0 or positive and has a value ranging from 0 to 20;
the subscript m is 0 or positive and has a value ranging from 0 to 200;
the subscript q is 0 or positive and has a value ranging from 0 to 30;
the subscript j is 0 or positive and has a value ranging from 0 to 2;
the subscript n is 0 or positive and has a value ranging from 0 to 20;
the subscript r is 0 or positive and has a value ranging from 0 to 30;
the subscript t is 0 or 1;
the subscript u is 0 or 1;
the subscript v is 0 or positive and has a value ranging from 0 to 100 subject to the limitation that $(v+w+x) > 0$;
the subscript w is 0 or positive and has a value ranging from 0 to 100 subject to the limitation that $(v+w+x) > 0$; and
the subscript x is 0 or positive and has a value ranging from 0 to 100 subject to the limitation that $(v + w + x) > 0$.

Alternatively, the siloxane comprises an oxirane or oxetane compound which is a hydrocarbon having the formula:

$$R^{22}_y(R^{23})_z(R^{24}_\alpha)(R^{25})_\beta$$

where $R^{22}$ and $R^{25}$ are independently a monovalent hydrocarbon radical containing one or more oxirane or oxetane moieties having from 3 to 12 carbon atoms;
$R^{23}$ and $R^{24}$ are each selected from the group consisting of H or a linear or branched monovalent hydrocarbon radical of 1 to 200 carbons;

the subscripts y, z, $\alpha$, $\beta$ are zero or positive ranging from zero to four subject to the limitation that $(y + \beta) > 2$ or alternatively where the oxirane or oxetane compound is a polyether having the formula:

$$R^{26}O(R^{27})_{\gamma}(C_2H_4O)_{\delta}(C_3H_6O)_{\varepsilon}(C_4H_8O)_{\zeta}R^{28}$$

where $R^{26}$ and $R^{28}$ are independently a monovalent hydrocarbon radical containing one or more oxirane or oxetane moieties having from 3 to 12 carbon atoms;
$R^{27}$ is selected from the group of divalent radicals consisting of $-C_2H_4O-$, $-C_3H_6O-$, and $-C_4H_8O-$; the subscript $\gamma$ is zero or 1;
the subscript $\delta$ is zero or positive and has a value ranging from 0 to 100 subject to the limitation that $(\delta + \varepsilon + \zeta) > 0$;
the subscript $\varepsilon$ is zero or positive and has a value ranging from 0 to 100 subject to the limitation that $(\delta + \varepsilon + \zeta) > 0$;
the subscript $\zeta$ is zero or positive and has a value ranging from 0 to 100 subject to the limitation that $(\delta + \varepsilon + \zeta) > 0$.

The aminosilane has the formula:

$$N(H)(R^1)R^2Si(OR^3)_{3-b-c}(OR^4)_a(R^5Si(OR^6)_d(R^7)_e)_bR^8_c$$

with

$R^1$ is chosen from the group consisting of H or a monovalent hydrocarbon radical containing 1 to 20 carbon atoms;
$R^2$ is selected from a group consisting of a divalent linear or branched hydrocarbon radical consisting of 1 to 60 carbons;
$R^4$ is a hydrocarbon radical that contains 3 to 200 carbon atoms;
$R^5$ is selected from a group consisting of oxygen or a divalent linear or branched hydrocarbon radical consisting of 1 to 60 carbons;
$R^3$, $R^6$, $R^7$, and $R^8$ and are each independently selected from the group of monovalent linear or branched hydrocarbon radicals having from 1 to 200 carbon atoms;
the subscript b is 0 or a positive number and has a value ranging from 0 to 3;
the subscript a is a positive number less than 3;
the subscripts b and c are 0 or positive and have a value ranging from 0 to 3 subject to the limitation that $(a+b+c) \leq 3$; and
the subscripts d and e are 0 or positive and have a value ranging from 0 to 3 subject to the limitation that $(d+e) = 3$.

The film-forming aminosilicone polymer of the hair coloring composition may be the product of the reaction of the siloxane (a1), the aminosilane (b1), and a compound (I) having the formula:

$$\text{(I)} \qquad R^{29}(R^{30})_{\kappa}Si(OR^{31})_{3-\eta-\theta}(R^{32})_{\eta}(OR^{33})_{\theta}$$

where

$R^{29}$ is a monovalent hydrocarbon radical containing one or more oxirane or oxetane moieties having from 3 to 12 carbon atoms;
$R^{30}$ is a divalent hydrocarbon radical consisting of 1 to 60 carbons and the subscript $\kappa$ has a value of 0 or 1; $R^{31}$ and $R^{32}$ are independently selected from the group of monovalent linear or branched hydrocarbon radicals having from 1 to 60 carbon atoms;
the subscript $\eta$ is zero or positive and has a value ranging from 0 to 3;
the subscript $\theta$ is greater than 0 and less than or equal to 3, subject to the limitation that $3-\eta-\theta$ is greater than or equal to 0; and
$R^{33}$ is a hydrocarbon radical that contains 3 to 200 carbon atoms.

As used herein the phrase hydrocarbon radical includes hydrocarbon radicals that may be optionally substituted with hetero-atoms particularly nitrogen, oxygen, and sulfur, and may optionally contain ring structures such as oxirane and oxetane groups.

The hair coloring composition may comprise:

the aminosilane wherein $R^1$ has from 1 to 5 carbon atoms; $R^2$ has from 2 to 8 carbon atoms; $R^4$ has from 3 to 8 carbon atoms; $R^3$, $R^6$, $R^7$, and $R^8$ each independently have from 1 to 15 carbon atoms; the subscript a ranges from 2 to 3; the subscript b ranges from 0 to 15; the subscript c ranges from 0 to 2;
the siloxane wherein $R^9$, $R^{10}$, $R^{11}$, $R^{12}$, $R^{13}$, $R^{18}$, $R^{19}$, $R^{20}$, and $R^{21}$ each independently have from 1 to 3 carbon atoms; the subscript k ranges from 5 to 250; the subscripts v, w, and x each independently range from 0 to 35; and

the compound (I) wherein $R^{31}$ and $R^{32}$ each independently have from 1 to 8 carbon atoms, and $R^{33}$ has from 3 to 50 carbon atoms.

The hair coloring composition may comprise:

the aminosilane wherein $R^1$ is H; $R^2$ has from 2 to 5 carbon atoms; $R^4$ has from 3 to 5 carbon atoms; $R^3$, $R^6$, $R^7$, and $R^8$ each independently have from 2 to 8 carbon atoms; the subscript a ranges from 0 or 1; the subscript b is 3; the subscript c is 0 or 1;
the siloxane wherein $R^9$, $R^{10}$, $R^{11}$, $R^{12}$, $R^{13}$, $R^{18}$, $R^{19}$, $R^{20}$, and $R^{21}$ are each independently methyl; the subscript k ranges from 5 to 150; the subscripts v, w, and x each independently range from 0 to 25; and
the compound (I) wherein $R^{31}$ and $R^{32}$ each independently have from 1 to 4 carbon atoms; and $R^{33}$ has from 3 to 10 carbon atoms.

The hair coloring composition may preferably comprise wherein:

the aminosilane wherein $R^1$ is H; $R^2$ has from 2 to 5 carbon atoms; $R^3$ has from 3 to 5 carbon atoms; $R^4$ has from 3 to 5 carbon atoms; $R^3$, $R^6$, $R^7$, and $R^8$ each independently have from 2 to 8 carbon atoms; the subscript a is 1; the subscript b is 0; the subscript c is 0; and
the siloxane wherein $R^9$, $R^{10}$, $R^{11}$, $R^{12}$, $R^{13}$, $R^{18}$, $R^{19}$, $R^{20}$, and $R^{21}$ are each independently methyl; the subscript k ranges from 5 to 150; the subscripts v, w, and x each independently range from 0 to 25; and the subscript n is 0.

The hair coloring composition may more preferably comprise the siloxane which is an epoxy encapped polysiloxane with the average structure:

$$CH_2(O)CHCH_2O(CH_2)_\lambda Si(CH_3)_2O[Si(CH_3)_2O]_kSi(CH_3)_2CH_2CH_2CH_2OCH_2CH(O)CH_2$$

with the subscript k ranges from 5 to 250, from 5 to 150, preferably from 50 to 100, and the subscript $\lambda$ is 2 or 3; and the aminosilane which has the formula $H_2NR^2Si(OR^3)_2(OR^4)$ with
$R^2$ has from 2 to 5 carbon atoms;
$R^3$ has from 3 to 5 carbon atoms; and
$R^4$ has from 3 to 5 carbon atoms.

The hair coloring composition may even more preferably comprise as the siloxane a glycidoxypropyl-terminated dimethyl siloxane polymer with the average structure $CH_2(O)CHCH_2O(CH_2)_3Si(CH_3)_2O[Si(CH_3)_2O]_{50}Si(CH_3)_2CH_2CH_2CH_2OCH_2CH(O)CH_2$, and as the aminosilane the aminopropyltriisopropoxysilane.

The hair coloring composition may even more preferably comprise the film-forming aminosilicone polymer which is the product of the reaction of the siloxane (a1), the aminosilane (b1), and an encapped polyether with the average structure $CH_2(O)CHCH_2O(CH_2CH_2O)_uCH_2CH(O)CH_2$ with the subscript $\beta$ ranges from 10 to 20, or an encapped polyether with the average structure $CH_2(O)CHCH_2O(CH_2CH_2O)_u(CH_2CHCH_3O)_vCH_2CH(O)CH_2$ with the subscript $\mu$ ranges from 1 to 20 and with the subscript v ranges from 1 to 20.

The hair coloring composition may even more preferably comprise as the siloxane a glycidoxypropyl-terminated dimethyl siloxane polymer which is the epoxy encapped poly siloxane with the average structure $CH_2(O)CHCH_2O(CH_2)_3Si(CH_3)_2O[Si(CH_3)_2O]_{50}Si(CH_3)_2CH_2CH_2CH_2OCH_2CH(O)CH_2$, and as the aminosilane the aminopropyltriisopropoxysilane, and as the encapped polyether PEG-13 diglycidyl ether, and optionally diethylaminopropylamine.

In reacting the siloxane (oxirane or oxetane) compounds with amino bearing compounds, the mole ratio of oxirane or epoxy groups to amino groups may be preferably 1 to 4, more preferably greater than 1.1 and less than 3.9, and most preferably greater than 1.2 and less than 3.8. $R^1$ may be preferably a monovalent hydrocarbon radical of from 1 to 10 carbon atoms or hydrogen, more preferably from 1 to 5 carbon atoms or hydrogen, most preferably $R^1$ may be H. $R^2$ may be preferably a monovalent hydrocarbon radical of from 1 to 10 carbon atoms more preferably 2 to 8 carbon atoms, and most preferably 3 to 5 carbon atoms. $R^4$ may be preferably a monovalent hydrocarbon radical of from 3 to 10 carbon atoms, more preferably 3 to 8 carbon atoms, most preferably 3 to 5 carbon atoms. $R^3$, $R^6$, $R^7$, and $R^8$ may be each preferably a monovalent hydrocarbon radical of from 1 to 20 carbon atoms, more preferably 1 to 15 carbon atoms, most preferably 2 to 8 carbon atoms. Subscript a may be in the range of from 0 to 3, preferably from 1 to 3, more preferably from 2 to 3, most preferably from 0 to 1. Subscript b may be in the range of 0 to 25, more preferably 0 to 15 and most preferably 3. Subscript c may be in the range 0 to 3, more preferably 0 to 2, most preferably 0 to 1. $R^9$, $R^{10}$,

$R^{11}$, $R^{12}$, $R^{13}$, $R^{18}$, $R^{19}$, $R^{20}$, and $R^{21}$ may be each preferably a monovalent hydrocarbon radical of from 1 to 4 carbon atoms, more preferably 1 to 3 carbon atoms, and most preferably 1 carbon atom. The subscripts f, 1, m, n, o, p, q, r, s may be each in the range of 0 to 200, more preferably 0 to 100, and most preferably 0 to 50. The subscript k may be in the range of 0 to 500, more preferably 5 to 250, and most preferably 5 to 150. The subscripts v, w, and x may be each in the range of 0 to 50, more preferably 0 to 35, and most preferably 0 to 25. $R^{23}$ and $R^{24}$ may be each preferably a monovalent hydrocarbon radical of from 5 to 1000 carbon atoms, more preferably 10 to 500, and most preferably 10 to 300. The subscripts $\delta$, $\varepsilon$, $\zeta$ may be in the range of 0 to 50 more preferably, 0 to 30, and most preferably 0 to 15. $R^{31}$ and $R^{32}$ may be each preferably a monovalent hydrocarbon radical of from 1 to 10 carbon atoms, more preferably 1 to 8 carbon atoms, and most preferably 1 to 4 carbon atoms. $R^{33}$ may be each preferably a monovalent hydrocarbon radical of from 3 to 100 carbon atoms, more preferably 3 to 50 carbon atoms, most preferably 3 to 10 carbon atoms.

Reference is made to substances, components, or ingredients in existence at the time just before first contacted, formed in situ, blended, or mixed with one or more other substances, components, or ingredients in accordance with the present disclosure. A substance, component or ingredient identified as a reaction product, resulting mixture, or the like may gain an identity, property, or character through a chemical reaction or transformation during the course of contacting, in situ formation, blending, or mixing operation if conducted in accordance with this disclosure with the application of common sense and the ordinary skill of one in the relevant art (e.g., chemist). The transformation of chemical reactants or starting materials to chemical products or final materials is a continually evolving process, independent of the speed at which it occurs. Accordingly, as such a transformative process is in progress there may be a mix of starting and final materials, as well as intermediate species that may be, depending on their kinetic lifetime, easy or difficult to detect with current analytical techniques known to those of ordinary skill in the art.

Reactants and components referred to by chemical name or formula in the specification or claims hereof, whether referred to in the singular or plural, may be identified as they exist prior to coming into contact with another substance referred to by chemical name or chemical type (e.g., another reactant or a solvent). Preliminary and/or transitional chemical changes, transformations, or reactions, if any, that take place in the resulting mixture, solution, or reaction medium may be identified as intermediate species, master batches, and the like, and may have utility distinct from the utility of the reaction product or final material. Other subsequent changes, transformations, or reactions may result from bringing the specified reactants and/or components together under the conditions called for pursuant to this disclosure. In these other subsequent changes, transformations, or reactions the reactants, ingredients, or the components to be brought together may identify or indicate the reaction product or final material.

In describing the products of the instant invention as a reaction product of initial materials reference is made to the initial species recited and it is to be noted that additional materials may be added to the initial mixture of synthetic precursors. These additional materials may be reactive or non-reactive. The defining characteristic of the instant invention is that the reaction product is obtained from the reaction of at least the components listed as disclosed. Non-reactive components may be added to the reaction mixture as diluents or to impart additional properties unrelated to the properties of the composition prepared as a reaction product. Additional reactive components may also be added; such components may react with the initial reactants or they may react with the reaction product; the phrase "reaction product" is intended to include those possibilities as well as including the addition of non-reactive components.

Optionally, the film-forming aminosilicone polymer of the hair coloring composition may be the product of the reaction of the siloxane and the aminosilane in the presence of a primary or a secondary amine.

The primary amine may be preferably selected from the group consisting of, methylamine, ethylamine, propylamine, ethanol amine, isopropylamine, butylamine, isobutylamine, hexylamine, dodecylamine, oleylamine, aniline aminopropyltrimethylsilane, aminopropyltriethylsilane, aminomorpholine, 3-(diethylamino)propylamine, benzylamine, napthylamine 3-amino-9-ethylcarbazole, 1 -amino heptaphlorohexane, 2,2,3,3,4,4,5,5,6,6,7,7,8,8,8-pentadecafluoro-1-octanamine, and mixtures thereof. The primary amine may be more preferably 3-(diethylamino)propylamine.

Alternatively, the secondary amine may be selected from the group consisting of methylethylamine, methylhexylamine, methyloctadecylamine, diethanolamine, dibenzylamine, dihexylamine, dicyclohexylamine, piperidine, pyrrolidine, phthalimide, and mixtures thereof.

Suitable example film-forming aminosilicone polymers can be found in the following European patent EP 2 214 633 B1 in par. 40-51.

The hair coloring composition may comprise from 1% to 20%, preferably from 1.5% to 15%, more preferably from 5% to 10% of a film-forming aminosilicone polymer, by total weight of the composition.

A suitable film-forming aminosilicone polymer for use in the present invention is available under the trade mark Silsoft® CLX-E by the company Momentive Performance Materials, Albany, New York, (US). Silsoft® CLX-E has as the INCI name "dipropylene glycol and polysilicone-19". Polysilicone-29 is a complex silicone polymer formed by the reaction between a glycidoxypropyl-terminated dimethyl siloxane polymer, PEG-13 diglycidyl ether, diethylaminopropylamine, and aminopropyltriisopropoxysilane.

Hair coloring agent

The hair coloring composition comprises a hair coloring agent. The hair coloring agent may be selected from the group consisting of pigment, coloured material, direct dye; and mixtures thereof. Alternatively, the hair coloring composition may be an oxidation dye.

Pigments

The hair coloring agent may comprise one or more pigments, preferably may consist essentially of one or more pigments. The one or more pigments may be one or more colored pigments which impart color effects to the product mass or to the hair. Alternatively, the one or more pigments may be lustre effect pigments which impart desirable and aesthetically pleasing lustre effects to the composition or to the keratin fibers. The color or lustre effects on the hair may be preferably temporary, i.e. they last until the next hair wash and can be removed again by washing the hair with customary shampoos.

The hair coloring agent may comprise one or more pigments having a $D_{50}$ particle diameter of from 5 micron to 60 micron. Particle diameter is represented by $D_{50}$, which is the median diameter by volume. $D_{50}$ is measured with a Malvern Mastersizer 2000, which is a laser diffraction particle sizer and it is measured according to ISO 13320:2009(en) with Hydro 2000G or Hydro 2000S where the dispersant is water or ethanol. Detection range is from 0.02 micron to 2000 micron. $D_{50}$ is expressed as $x_{50}$ in ISO 13320:2009(en). Laser diffraction measures particle size distributions by measuring the angular variation in intensity of light scattered as a laser beam passes through a dispersed particulate sample analyser and the particle size is reported as a volume equivalent sphere diameter. A discussion of calculating $D_{50}$ is provided in Barber et al, Pharmaceutical Development and Technology, 3(2), 153-161 (1998).

The hair coloring agent may comprise one or more pigments having a $D_{50}$ particle diameter of from 10 micron to 40 micron. The one or more pigments may be present in the hair coloring composition in an undissolved form. The hair coloring composition may comprise from 0.01% to 25%, or from 0.1% to 20% of one or more pigments, or from 1% to 15%, or from 4% to 10% of the one or more pigments, by total weight of the composition.

The one or more pigments may be one or more colorants which are virtually insoluble in the hair coloring composition, and may be inorganic or organic. Inorganic-organic mixed pigments may be also possible. The hair coloring agent may comprise one or more inorganic pigments. The advantage of the one or more inorganic pigments is their excellent resistance to light, weather and temperature. The one or more inorganic pigments may be of natural origin, and are, for example, derived from material selected from the group consisting of chalk, ochre, umber, green earth, burnt sienna, and graphite.

The one or more pigments may be one or more white pigments, such as, for example, titanium dioxide or zinc oxide, or may be one or more black pigments, such as, for example, iron oxide black, or may be one or more colored pigments, such as, for example, ultra-marine or iron oxide red, one or more lustre pigments, one or more metal effect pigments, one or more pearlescent pigments, and one or more fluorescent or one or more phosphorescent pigments.

The one or more pigments may be one or more colored, non-white pigments. The one or more pigments may be selected from the group consisting of metal oxides, hydroxides and oxide hydrates, mixed phase pigments, sulfur-containing silicates, metal sulfides, complex metal cyanides, metal sulfates, chromates and molybdates, and the metals themselves (bronze pigments). The one or more pigments may be selected from the group consisting of are titanium dioxide (CI 77891), black iron oxide (CI 77499), yellow iron oxide (CI 77492), red and brown iron oxide (CI 77491), manganese violet (CI 77742), ultramarine (sodium aluminium sulfosilicates, CI 77007, Pigment Blue 29), chromium oxide hydrate (CI 77289), Prussian blue (ferric ferrocyanide, CI 77510), carmine (cochineal), and combinations thereof.

The one or more pigments may be one or more pearlescent and colored pigments based on mica which are coated with a metal oxide or a metal oxychloride, such as titanium dioxide or bismuth oxychloride, and optionally further color-imparting substances, such as iron oxides, Prussian blue, ultramarine, and carmine. The color exhibited by the one or more pigments can be adjusted by varying the layer thickness. Such pigments are sold, for example, under the trade names Rona®, Colorona®, Dichrona®, RonaFlair®, Ronastar®, Xirona® and Timiron® all of which are available from Merck, Darmstadt, Germany. For example, Xirona® is a brand for color travel pigments that display color shifting effects depending on the viewing angle and are based on either natural mica, silica (SiO2) or calcium aluminium borosilicate flakes, coated with varying layers of titanium dioxide ($TiO_2$).

Pigments from the line KTZ® from Kobo Products, Inc., 3474 So. Clinton Ave., So. Plainfield, USA, are also useful herein, in particular the Surface Treated KTZ® Pearlescent Pigments from Kobo. Particularly useful are KTZ® FINE WHITE (mica and $TiO_2$) having a $D_{50}$ particle diameter of 5 to 25 micron and also KTZ® CELESTIAL LUSTER (mica and $TiO_2$, 10 to 60 micron) as well as KTZ® CLASSIC WHITE (mica and $TiO_2$, 10 to 60 micron). Also useful are SynCrystal Sapphire from Eckart Effect Pigments, which is a blue powder comprising platelets of synthetic fluorphlogopite coated with titanium dioxide, ferric ferrocyanide and small amounts of tin oxide. Also useful is SYNCRYSTAL Almond also from Eckart, which is a beige powder with a copper reflection color and is composed of platelets of synthetic

fluorphlogopite and coated with titanium dioxide and iron oxides. Also useful is Duocrome® RV 524C from BASF, which provides a two color look via a lustrous red powder with a violet reflection powder due to its composition of mica, titanium dioxide and carmine.

The one or more pigments may be one or more organic pigments. The one or more organic pigments may be selected from the group consisting of natural pigments sepia, gamboge, bone charcoal, Cassel brown, indigo, chlorophyll and other plant pigments.

The one or more organic pigments may be one or more synthetic organic pigments. The one or more synthetic organic pigments may be selected from the group consisting of azo pigments, anthraquinoids, indigoids, dioxazine, quinacridone, phthalocyanine, isoindolinone, perylene and perinone, metal complex, alkali blue, diketopyrrolopyrrole pigments, and combinations thereof.

The one or more pigments may be selected from the group consisting of iron oxide, titanium dioxide, mica, borosilicate, and combinations thereof. The one or more pigments may comprise one or more iron oxide ($Fe_2O_3$) pigments. The one or more pigments may comprise a combination of mica and titanium dioxide.

Colored material

The hair coloring agent may comprise one or more colored materials, preferably may consist essentially of one or more colored materials. The one or more colored materials may be particulate in form. The one or more colored materials may be selected from the group consisting of colored fibers, colored beads, colored particles such as nano-particles, colored polymers comprising covalently attached dyes, liquid crystals, particles having diffraction properties, UV absorber and photoprotective substances, pressure- or light-sensitive pigments, and combinations thereof.

The one or more colored materials may be capable of changing color via a mechanism selected from the group consisting of thermochromism, photochromism, hydrochromism, magnetochromism, electrochromism, piezochromism, chemichromism, mechano-optics. Suitable materials may include 3D Magnetic Pigments, Glow Dust, Fluorescent Pigments, Thermo Dust, Chameleon Pigments and other color changing materials from Solar Color Dust (http://solarcolordust.com/).

The hair coloring agent may comprise one or more photoprotective substances. The hair coloring composition may comprise from 0.01% to 10%, or from 0.1% to 5%, or from 0.2% to 2% of the one or more photoprotective substances, by total weight of the composition. Useful photoprotective substances are specified in EP1084696A1 from §0036 to §0053. The one or more photoprotective substances may be selected from the group consisting of 2-ethylhexyl 4-methoxy-cinnamate, methyl methoxycinnamate, 2-hydroxy-4-methoxybenzophenone-5-sulfonic acid, polyethoxylated p-aminobenzoates, di-butyl-hydroxytoluene (BHT), and mixtures thereof.

The hair coloring composition may comprise from 0.01% to 10%, or from 0.05% to 5% of one or more particulate substances, by total weight of the composition. The one or more particulate substances may be substances which are solid at room temperature (23°C) and may be in the form of particles. The one or more particulate substances may be selected from the group consisting of silica, silicates, aluminates, clay earths, mica, and insoluble salts. The one or more particulate substances may be selected from the group consisting of insoluble inorganic metal salts, metal oxides, minerals and insoluble polymer particles. The one or more particulate substances may be titanium dioxide.

The one or more particulate substances may be present in the hair coloring composition in undissolved, or stably dispersed form, and, following application to the hair and evaporation of the solvent, can deposit on the hair in solid form.

The one or more particulate substances may be selected from the group consisting of silica (silica gel, silicon dioxide) and metal salts, in particular inorganic metal salts. The one or more particulate substances may be silica. The one or more particulate substances may be selected from the group consisting of metal salts such as alkali metal or alkaline earth metal halides, e.g. sodium chloride or potassium chloride; alkali metal or alkaline earth metal sulfates, such as sodium sulfate or magnesium sulfate.

Direct dyes

The hair coloring agent may comprise one or more direct dyes, preferably may consist essentially of one or more direct dyes.

The hair coloring composition may comprise a total amount of from 0.001% to 4%, or from 0.005% to 3%, or from 0.01% to 2% of one or more direct dyes, by total weight of the composition. The presence of one or more direct dyes and the proportion thereof may be useful in that it can provide or enhance coloring/dyeing, particularly with regard to intensity.

The one or more direct dyes may be selected from the group consisting of nitro dyes to provide a blue color, nitro dyes to provide a red color, nitro dyes to provide a yellow color, quinone dyes, basic dyes, neutral azo dyes, acid dyes, and mixtures thereof. The one or more direct dyes may be a nitro dye to provide a blue color. The one or more

direct dyes may be a nitro dye to provide a red color. The one or more direct dyes may be a nitro dye to provide a yellow color. The one or more direct dyes may be a quinone dye. The one or more direct dyes may be a basic dye. The one or more direct dyes may be a neutral azo dye. The one or more direct dyes may be an acid dye.

The one or more direct dyes may be selected from the group consisting of Acid dyes such as Acid Yellow 1, Acid Orange 3, Acid Black 1, Acid Black 52, Acid Orange 7, Acid Red 33, Acid Yellow 23, Acid Blue 9, Acid Violet 43, Acid Blue 16, Acid Blue 62, Acid Blue 25, Acid Red 4, Basic Dyes such as Basic Brown 17, Basic Red 118, Basic Orange 69, Basic Red 76, Basic Brown 16, Basic Yellow 57, Basic Violet 14, Basic Blue 7, Basic Blue 26, Basic Red 2, Basic Blue 99, Basic Yellow 29, Basic Red 51, Basic Orange 31, Basic Yellow 87, 4-(3-(4-amino-9,10-dioxo-9,10-dihydroanthracen-1-ylamino)propyl)-4-methylmorpholin-4-ium-methylsulfate, (E)-1-(2-(4-(4,5-dimethylthiazol-2-yl)diazenyl)phenyl)(ethyl)amino)ethyl)-3-methyl-1H-imidazo1-3-ium chloride, (E)-4-(2-(4-(dimethylamino)phenyl)diazenyl)-1-methyl-1H-imidazol-3-ium-3-yl)butane-1-sulfonate, (E)-4-(4-(2-methyl-2-phenylhydrazono)methyl)pyridinium-1-yl)butane-1-sulfonate, N,N-dimethyl-3-(4-(methylamino)-9,10-dioxo-4a,9,9a,10-tetrahydroanthracen-1-ylamino)-N-propylpropan-1-aminium bromide, Disperse Dyes such as Disperse Red 17, Disperse Violet 1, Disperse Red 15, Disperse Violet 1, Disperse Black 9, Disperse Blue 3, Disperse Blue 23, Disperse Blue 377, Nitro Dyes such as 1-(2-(4-nitrophenylamino)ethyl)urea, 2-(4-methyl-2-nitrophenylamino)ethanol, 4-nitrobenzene-1,2-diamine, 2-nitrobenzene-1,4-diamine, Picramic acid, HC Red No. 13, 2,2'-(2-nitro-1,4-phenylene)bis(azanediyl)diethanol, HC Yellow No. 5, HC Red No. 7, HC Blue No.2, HC Yellow No. 4, HC Yellow No. 2, HC Orange No. 1, HC Red No. 1, 2-(4-amino-2-chloro-5-nitrophenylamino)ethanol, HC Red No. 3, 4-amino-3-nitrophenol, 4-(2-hydroxyethylamino)-3-nitrophenol, 2-amino-3-nitrophenol, 2-(3-(methylamino)-4-nitrophenoxy)ethanol, 3-(3-amino-4-nitrophenyl)propane-1,2-diol, HC Yellow No. 11, HC Violet No. 1, HC Orange No. 2, HC Orange No. 3, HC Yellow No. 9, HC Red No. 10, HC Red No. 11, 2-(2-hydroxyethylamino)-4,6-dinitrophenol, HC Blue No. 12, HC Yellow No. 6, HC Yellow No. 12, HC Blue No. 10, HC Yellow No. 7, HC Yellow No. 10, HC Blue No. 9, 2-chloro-6-(ethylamino)-4-nitrophenol, 6-nitropyridine-2,5-diamine, HC Violet No. 2, 2-amino-6-chloro-4-nitrophenol, 4-(3-hydroxypropylamino)-3-nitrophenol, HC Yellow No. 13, 6-nitro-1,2,3,4-tetrahydroquinoxaline, HC Red No. 14, HC Yellow No. 15, HC Yellow No. 14, N-2-methyl-6-nitropyridine-2,5-diamine, N1-allyl-2-nitrobenzene-1,4-diamine, HC Red No. 8, HC Green No.1, HC Blue No. 14, and Natural dyes such as Annato, Anthocyanin, Beetroot, Carotene, Capsanthin, Lycopene, Chlorophyll, Henna, Indigo, Cochineal.

Oxidation dye

The hair coloring agent may comprise a dye composition and optionally a developer composition. The developer composition may comprise one or more oxidizing agents. The dye composition may comprise one or more oxidative dye precursors comprising one or more couplers and one or more primary intermediates or the dye composition may consist essentially of one or more direct dyes.

Oxidative dye precursors

The dye composition according to the present invention may comprise oxidative dye precursors, which are usually classified either as primary intermediates (also known as developers) or couplers (also known as secondary intermediates). Various couplers may be used with primary intermediates in order to obtain different shades. Oxidative dye precursors may be free bases or the cosmetically acceptable salts thereof.

Hence, the dye composition may comprise oxidative dyes precursors comprising one or more couplers (also known as secondary intermediate) and one or more primary intermediates (also known as developer).

The oxidative dye precursors suitable for use herein, in so far as they are bases, may be used as free bases or in the form of any cosmetically acceptable salts obtained with the corresponding organic or inorganic acids, such as hydrochloric, hydrobromic, citric, acetic, lactic, succinic, tartaric, or sulfuric acids, or, in so far as they have aromatic hydroxyl groups, in the form of any cosmetically acceptable salts obtained with the corresponding bases, such as alkali phenolates.

Oxidative dye precursors are known in the art, and include aromatic diamines, aminophenols, aromatic diols and their derivatives (a representative but not exhaustive list of oxidation dye precursors can be found in Sagarin, "Cosmetic Science and Technology", Interscience, Special Edn. Vol. 2 pages 308 to 310). Suitable oxidative dye precursors are also disclosed in the Canadian Patent Application No. CA2576189A1 - in particular, from Table 1 dye combinations No. 1 to 2394, which span pages 49 to 238. It is to be understood that the one or more primary intermediates and the one or more couplers (collectively known as oxidative dye precursors) detailed below are only by way of example and are not intended to limit the compositions and other aspects herein described. The one or more primary intermediates and the one or more couplers may be used in the form of any cosmetically acceptable salts, for example sulfate salts.

Typically, the dye composition may comprise a total amount of oxidative dye precursors ranging up to 12%, from 0.001% to 12%, preferably from 0.1% to 10%, more preferably from 0.3% to 8%, even more preferably from 0.5% to 6%, by total weight of the composition.

The one or more primary intermediates may be selected from the group consisting of toluene-2,5-diamine, p-phenylenediamine, N-phenyl-p-phenylenediamine, N,N-bis(2-hydroxyethyl)-p-phenylenediamine, 2-hydroxyethyl-p-phenylenediamine, hydroxypropyl-bis-(N-hydroxyethyl-p-phenylenediamine), 2-methoxymethyl-p-phenylenediamine, 2-(1,2-dihydroxyethyl)-p-phenylenediamine, 2,2'-(2-(4-aminophenylamino)ethylazanediyl)diethanol, 2-(2,5-diamino-4-methoxyphenyl)propane-1,3-diol, 2-(7-amino-2H-benzo[b][1,4]oxazin-4(3H)-yl)ethanol, 2-chloro-p-phenylenediamine, p-aminophenol, p-(methylamino)phenol, 4-amino-m-cresol, 6-amino-m-cresol, 5-ethyl-o-aminophenol, 2-methoxy-p-phenylenediamine, 2,2'-methylenebis-4-aminophenol, 2,4,5,6-tetraminopyrimidine, 2,5,6-triamino-4-pyrimidinol, 1-hydroxyethyl-4,5-diaminopyrazole sulfate, 4,5-diamino-1-methylpyrazole, 4,5-diamino-1-ethylpyrazole, 4,5-diamino-1-isopropylpyrazole, 4,5-diamino-1-butylpyrazole, 4,5-diamino-1-pentylpyrazole, 4,5-diamino-1-benzylpyrazole, 2,3-diamino-6,7-dihydropyrazolo[1,2-a]pyrazol-1(5H)-one dimethosulfonate, 4,5-diamino-1-hexylpyrazole, 4,5-diamino-1-heptylpyrazole, methoxymethyl-1,4-diaminobenzene, N,N-bis(2-hydroxyethyl)-N-(4-aminophenyl)-1,2-diaminothane, 2-[(3-aminopyrazolo[1,5-a]pyridin-2-yl)oxy]ethanol hydrochloride, their salts thereof and mixtures thereof.

The one or more primary intermediates of the dye composition may be particularly 1,4-diamino-2-(methoxymethyl)-benzene. 1,4-diamino-2-(methoxymethyl)-benzene has the advantage of an improved sensitisation profile (i.e. reduced risks of scalp skin reaction).

The one or more primary intermediates may be 4,5-diamino-1-hexylpyrazole. 4,5-diamino-1-hexylpyrazole may be used as a sulfate salt.

The one or more primary intermediates may be selected from the group consisting of 4,5-diamino-1-butylpyrazole, 4,5-diamino-1-pentylpyrazole, 4,5-diamino-1-benzylpyrazole, 2,3-diamino-6,7-dihydropyrazolo[1,2-a]pyrazo1-1(5H)-one dimethosulfonate, 4,5-diamino-1-hexylpyrazole, 4,5-diamino-1-heptylpyrazole, methoxymethyl-1,4-diaminobenzene, and mixtures thereof; and the cosmetically acceptable salts thereof such as chlorides, sulfates and hemi-sulfates in particular.

The one or more couplers may be a compound comprising one or more phenyl rings substituted with one or more hydroxyl groups.

The one or more couplers may be selected from the group consisting of resorcinol, 4-chlororesorcinol, 2-chlororesorcinol, 2-methylresorcinol, 4,6-dichlorobenzene-1,3-diol, 2,4-dimethylbenzene-1,3-diol, m-aminophenol, 4-amino-2-hydroxytoluene, 2-methyl-5-hydroxyethylaminophenol, 3-amino-2,6-dimethylphenol, 3-amino-2,4-dichlorophenol, 5-amino-6-chloro-o-cresol, 5-amino-4-chloro-o-cresol, 6-hydroxybenzomorpholine, 2-amino-5-ethylphenol, 2-amino-5-phenylphenol, 2-amino-5-methylphenol, 2-amino-6-methylphenol, 2-amino-5-ethoxyphenol, 5-methyl-2-(methylamino)phenol, 2,4-diaminophenoxyethanol, 2-amino-4-hydroxyethylaminoanisole, 1,3-bis-(2,4-diaminophenoxy)-propane, 2,2'-(2-methyl-1,3-phenylene)bis(azanediyl)diethanol, benzene-1,3-diamine, 2,2'-(4,6-diamino-1,3-phenylene)bis(oxy)diethanol, 3-(pyrrolidin-1-yl)aniline, 1-(3 -(dimethylamino)phenyl)urea, 1-(3-aminophenyl)urea, 1-naphthol, 2-methyl-1-naphthol, 1,5-naphthalenediol, 2,7-naphthalenediol or 1-acetoxy-2-methylnaphthalene, 4-chloro-2-methylnaphthalen-1-ol, 4-methoxy-2-methylnaphthalen-1-ol, 2,6-dihydroxy-3,4-dimethylpyridine, 2,6-dimethoxy-3,5-pyridinediamine, 3-amino-2-methylamino-6-methoxypyridine, 2-amino-3-hydroxypyridine, 2,6-diaminopyridine, pyridine-2,6-diol, 5,6-dihydroxyindole, 6-hydroxyindole, 5,6-dihydroxyindoline, 3-methyl-1-phenyl-1*H*-pyrazol-5(4*H*)-one, 1,2,4-trihydroxybenzene, 2-(benzo[d][1,3]dioxol-5-ylamino)ethanol (also known as hydroxyethyl-3,4-methylenedioxyaniline), and mixtures thereof.

The oxidative dye precursors may be particularly selected from the group consisting of 1-naphthol, 2,4-diaminophenoxyethanol, toluene-2,5-diamine sulfate, resorcinol, 4-amino-m-cresol, 2-amino-6-chloro-4-nitrophenol, 2-amino-4-hydroxyethylaminoanisole sulfate, hydroxyethyl-3,4-methylenedioxyaniline HCl, 1-hydroxyethyl 4,5-diamino pyrazole sulfate, 4-amino-2-hydroxytoluene, 2-methylresorcinol, m-aminophenol, 2-methyl-5-hydroxyethylaminophenol, and mixtures thereof.

The oxidative dye precursors may comprise preferably 5-amino-4-chloro-o-cresol and 1,4-diamino-2-(methoxymethyl)-benzene. The oxidative dye precursors may comprise more preferably 2,6-diaminopyridine and 1,4-diamino-2-(methoxymethyl)-benzene. The oxidative dye precursors may comprise even more preferably 2,6-dihydroxyethylaminotoluene and 2-methoxymethyl-1,4-diaminobenzene. The oxidative dye precursors may comprise even more preferably 2-methoxymethyl-1,4-diaminobenzene and p-phenylenediamine and/or toluene-2,5-diamine.

When the hair coloring agent of the invention is obtained by mixing a dye composition and a developer composition, the primary intermediates and couplers are usually incorporated into the dye composition.

The dye composition of the hair coloring composition may further comprise beyond one or more oxidative dye precursors, one or more direct dyes, preferably one or more oxidatively stable direct dyes. Typically, the dye composition may comprise a total amount of direct dyes ranging from 0.001% to 4%, preferably from 0.005% to 3%, more preferably from 0.01% to 2%, by total weight of the composition.

Alternatively, the dye composition may preferably consist essentially of one or more direct dyes as set out hereinbefore.

The presence of one or more direct dyes and the proportion thereof can help to provide or enhance coloring/dyeing, particularly with regard to the vibrancy of the color that is desired.

The dye composition may be substantially free of any direct dyes. Indeed, sometimes consumers prefer direct dye-free compositions.

Oxidizing agent

The developer composition according to the present invention may comprise one or more oxidizing agent(s). The oxidizing agent(s) may preferably be selected from the group consisting of hypochlorous acid, peracetic acid, persulfate, preferably sodium persulfate, ammonium persulfate, chlorine dioxide, perboric acid, their salts thereof, ozone, hydrogen peroxide and mixtures thereof. The oxidizing agent(s) may more preferably be selected from the group consisting of hypochlorous acid, their salts thereof and mixtures thereof. The oxidizing agent(s) may even more preferably be selected from the group consisting of sodium hypochlorite, calcium hypochlorite, potassium hypochlorite and mixtures thereof.

The developer composition of the present invention may comprise one or more oxidizing agents and/or one or more sources of an oxidizing agent. The one or more oxidizing agents may be provided in aqueous solution or as a powder which is dissolved prior to use. Preferred oxidizing agents are water-soluble peroxygen oxidizing agents. Suitable water-soluble oxidizing agents include, but are not limited to: inorganic peroxygen materials capable of yielding hydrogen peroxide in an aqueous solution.

The one or more oxidizing agents are valuable for the initial solubilisation and decolorisation of the melanin (bleaching) and accelerate the oxidation of the oxidative dye precursors (oxidative dyeing) in the hair shaft.The developer composition may comprise a total amount of oxidizing agents ranging from 0.1% to 15%, alternatively from 0.2 % to 15%, alternatively from 0.3 % to 15%, alternatively ranging from 0.1% to 12%, alternatively from 0.2 % to 12%, alternatively from 0.3 % to 12%, alternatively from 0.1% to 7%, alternatively from 0.2% to 7%, alternatively from 0.3% to 7%, alternatively from 1% to 7%, alternatively from 0.1% to 5%, alternatively from 0.2% to 5%, alternatively from 0.3% to 5%, alternatively from 0.5% to 5%, alternatively from 1% to 5%, alternatively from 2% to 5%, by total weight of the composition.

Alternatively, the developer composition may comprise a total amount of oxidizing agents of less than 3%, alternatively less than 2%, alternatively less than 1%, alternatively less than 0.5%, alternatively less than 0.3% alternatively less than 0.1% by total weight of the composition. The lower limit for the oxidizing agents may be at least 0.01% by total weight of the composition.

The dye composition may also be substantially free of oxidizing agents, i.e. having oxidizing agents less than 0.1%, and more particularly less than 0.01% by total weight of the composition.

Suitable water-soluble peroxygen oxidizing agents include, but are not limited to: hydrogen peroxide; inorganic alkali metal peroxides (such as sodium periodate and sodium peroxide); organic peroxides (such as urea peroxide and melamine peroxide); inorganic perhydrate salt bleaching compounds (such as the alkali metal salts of perborates, percarbonates, perphosphates, persilicates, persulphates and the like); and mixtures thereof. Inorganic perhydrate salts may be incorporated for example as monohydrates, tetrahydrates. Alkyl/aryl peroxides and/or peroxidases may also be used. Mixtures of two or more such oxidizing agents can be used if desired.

The developer composition may comprise a water-soluble oxidizing agent selected from the group consisting of peroxides, percarbonates (which may be used to provide a source of both oxidizing agent and carbonate ions and or ammonium ions), persulphates, and mixtures thereof. The particularly preferred oxidizing agent is hydrogen peroxide.

When the hair coloring composition of the present invention is obtained by mixing a developer composition and a dye composition prior to use, the one or more oxidizing agents may be present in the developer composition. The developer composition may be based on any desired formulation chassis, including any commercial product, for example an oil-in-water emulsion. Typical developer compositions comprise 6% or 9% of the $H_2O_2$ relative to the total weight of the developer composition. A preferred example of a developer composition with respectively 6% and 9% $H_2O_2$, comprises as INCI ingredients: Water, $H_2O_2$, Cetearyl Alcohol, Ceteareth-25, Salicylic Acid, Phosphoric Acid, Disodium Phosphate, Etidronic Acid. Another preferred example a developer composition comprises as INCI ingredients: Water, $H_2O_2$, cetearyl alcohol, lanolin alcohol, sodium lauryl sulfate, parfum, salicylic acid, phosphoric acid, disodium phosphate, linalool, hexyl cinnamal, etidronic acid, tocopherol. Another preferred example a developer composition comprises as INCI ingredients: Water, $H_2O_2$, cetearyl alchohol, lanolin alcohol, sodium lauryl sulfate, parfum, salicylic acid, phosphoric acid, disodium phosphate, linalool, hexyl cinnamal, etidronic acid, tocopherol.

Optional ingredients for the hair coloring composition

The hair coloring composition according to the present invention may comprise, in addition to the ingredients indicated above, further ingredients in order to further enhance the properties of the hair coloring composition, as long as these are not excluded by the claims.

Suitable further optional ingredients include, in a cosmetically acceptable carrier, but not limited to: solvents; ether of water-soluble polyhydric alcohol, thickening system, anti-freeze agent; alkalizing agents; chelants; radical scavengers; pH modifiers and buffering agents; rheology modifiers; carbonate ion sources; peroxymonocarbonate ion sources; surfactants; perfume; enzymes; dispersing agents; peroxide stabilizing agents; antioxidants; natural ingredients (such as proteins, protein compounds, and plant extracts); conditioning agents (such as silicones and cationic polymers); ceramides; preservatives; opacifiers and pearling agents (such as titanium dioxide and mica); and mixtures thereof. The further optional ingredients may be present in the hair coloring composition in an amount ranging from 0% to 20% by weight, relative to the total weight of the hair coloring composition. Suitable further ingredients referred to above, but not specifically described below, are listed in the International Cosmetics Ingredient Dictionary and Handbook, (8th ed.; The Cosmetics, Toiletry, and Fragrance Association). Particularly, vol. 2, sections 3 (Chemical Classes) and 4 (Functions), which are useful in identifying specific adjuvants to achieve a particular purpose or multipurpose. A few of these ingredients are discussed hereinbelow, whose disclosure is of course non-exhaustive.

Ether of a water-soluble polyhydric alcohol

The hair coloring composition may comprise an ether of a water-soluble polyhydric alcohol. The ether of a water-soluble polyhydric alcohol may be selected from the group consisting of diethyleneglycol monobutylether, ethylene glycol monohexyl ether, and a mixture of diethyleneglycol monobutylether and ethylene glycol monohexyl ether. The composition may comprise from 0.01% to 20%, or from 0.1% to 10%, or from 0.5% to 5%, or from 1.0% to 5%, or from 2% to 5% ether of a water-soluble polyhydric alcohol by total weight of the composition.

Thickening system

The hair coloring composition may comprise a thickening system. The thickening system may comprise a deposition enhancer and a thickening polymer. The composition may comprise from 0.5% to 10% of a thickening system. The selected thickening system can help to prevent dripping of the hair coloring composition by inhibiting capillary action, which would otherwise quickly draw the hair coloring composition down the fibers and onto the floor and/or clothes of the user. Nevertheless, the thickening system, particularly the combination of thickening polymer and deposition enhancer, provide a non-dripping composition that adheres well to keratin fibers without generating a sticky feel. Moreover, the thickening system does not detract from the ability of the film-forming aminosilicone polymer to provide the thin, dry film.

The thickening system comprises a deposition enhancer. The deposition enhancer may be a hydrophilic and non-ionic polymer, and wherein the deposition enhancer has a weight average molecular weight of from 700,000 Dalton to 3,000,000 Dalton. The deposition enhancer may be useful for aiding the deposition of the film-forming aminosilicone polymer as well as any hair coloring agent on to the hair fiber. In particular, the deposition enhancer may have the advantage that more film-forming aminosilicone polymer as well as any hair coloring agent stays on the hair fiber rather than dripping or sliding off the hair fiber. Indeed, in view of the physical structure of a head of hair i.e. a plurality of fibers that are close proximity to one another, capillary action can play a role with regards to fluids on hair. Hence, the deposition enhancer can be useful for preventing the capillary action from stripping the film-forming aminosilicone polymer as well as any hair coloring agent from the hair.

The hair coloring composition may comprise from 0.01% to 10% of a deposition enhancer by total weight of the composition. The hair coloring composition may comprise from 0.05% to 4%, or from 0.075% to 3.5%, or from 0.1% to 3%, or from 0.1% to 2%, or from 0.15% to 1% of a deposition enhancer, by total weight of the composition.

The deposition enhancer may conform to the formula $H(OCH_2CH_2)_nOH$ wherein n has an average value of from 20,000 to 50,000. The deposition enhancer may conform to the formula $H(OCH_2CH_2)_nOH$ wherein n has an average value of from 40,000 to 50,000. The hair coloring composition may comprise from 0.01% to 10% of a deposition enhancer by total weight of the composition. The hair coloring composition may comprise from 0.05% to 4%, or from 0.075% to 3.5%, or from 0.1% to 3%, or from 0.1% to 2%, or from 0.15% to 1% of a deposition enhancer, by total weight of the composition, wherein the deposition enhancer conforms to the formula $H(OCH_2CH_2)_nOH$ wherein n has an average value of from 40,000 to 50,000.

Useful deposition enhancers are available from Dow under their POLYOX brand. In particular, POLYOX WSR N-60K is PEG-45M i.e. formula $H(OCH_2CH_2)_nOH$ wherein n is an integer and where n has an average value of 45,000. PEG-45M has a weight average molecular weight of 2,000,000 Dalton. Also useful is POLYOX WSR N-12K, which is PEG-23M i.e. formula $H(OCH_2CH_2)_nOH$ wherein n is an integer where n has an average value of 23,000. PEG-23M has a weight average molecular weight of 1,000,000 Dalton. Also useful is POLYOX WSR-1105, which has a weight average molecular weight of 900,000 Dalton.

The thickening system may comprise a thickening polymer. The thickening polymer may have a weight average molecular weight of at least 10,000 Dalton. The thickening polymer may be a cationic thickening polymer or is a non-ionic thickening polymer. The hair coloring composition may comprise from 0.01% to 10% of a thickening polymer by total weight of the composition. The hair coloring composition may comprise from 0.1%, or from 0.2%, or from 0.3%, or from 0.4, or from 0.5%, or from 0.6%, or from 0.7%, or from 0.8%, or from 0.9% to 5%, or to 4.5%, or to 4%, or to 3.5%,

or to 3%, or to 2.5%, or to 2%, or to 1.5% of a thickening polymer by total weight of the composition. The thickening polymer may be a non-ionic thickening polymer.

The thickening polymer may be a polysaccharide. The thickening polymer may be a polysaccharide and the polysaccharide may be selected from the group consisting of hydroxyethylcellulose, hydroxypropylcellulose, starch compounds, xanthan gum, carrageenans, and mixtures thereof. The thickening polymer may be a heteropolysaccharide. The total polysaccharide content present in the hair coloring composition may be from 0.2% to 5%, or from 0.5% to 4%, by total weight of the composition. Suitable polysaccharides and heteropolysaccharides may include starches and derivatives thereof, e.g. mono- or di-esters with phosphoric acid, cellulose types and their derivatives, xanthan gums, carrageenans. Heteropolysaccharides may include xanthan gum such as Keltrol® from Kelco, and Natrosol® 250 HHR from Herkules. The viscosity-increasing agent may be a starch compound. The viscosity-increasing agent may be a hydroxypropyl starch phosphate. An example of a hydroxypropyl starch phosphate may be Structure® XL from Akzo Nobel. The thickening polymer may be a hydroxethyl cellulose. A suitable hydroxethyl cellulose is Cellosize™ HEC QP 4400 from Dow.

The thickening polymer may be a cationic thickening polymer. The thickening polymer may comprise a hydrocarbon backbone substituted with an amino-group containing sidechain. The thickening polymer may be a cationic thickening polymer comprising a quaternary amine group, alternatively a quaternary ammonium group. The thickening polymer may be a Polyquaternium-37. Polyquaternium-37 is a poly(2-methacryloxyethyltrimethylammonium chloride). Polyquaternium-37 is available from BASF via the product Salcare® SC 96 FROM BASF, which has the INCI name: Polyquaternium-37 (and) Propylene Glycol Dicaprate Dicaprylate (and) PPG-1 Trideceth-6. Polyquaternium-37. Polyquaternium-37 is also available as: Syntran PC 5320 (Interpolymer Corporation); Ultragel® 300 from Cognis GmbH; OriStar PQ37 from Orient Stars LLC; Synthalen CN from 3V Group; Synthalen CR from 3V Group; Synthalen CU from 3V Group; Cosmedia® Triple C from Cognis GmbH, which has the INCI: Polyquaternium-37, Dicaprylyl Carbonate, Lauryl Glucoside.

Anti-freeze agent

The hair coloring composition may comprise from 0.5% to 30% of a volatile alcohol, wherein the volatile alcohol may have from 1 to 8 carbon atoms and may be miscible in water. The volatile alcohol may act as an anti-freeze agent (or a first anti-freeze agent). An anti-freeze agent has the advantage that it lowers the freezing point of a composition and consequently prevents the composition from freezing, which can prevent any unwanted side effects of freezing and/or subsequent thawing.

The hair coloring composition may comprise from 0.5% to 3%, or from 0.75% to 2.5%, or from 1% to 2% of a volatile alcohol by total weight of the composition, wherein the volatile alcohol may have from 1 to 8 carbon atoms and may be miscible in water. The volatile alcohol may be a monohydric alkyl alcohol having from 1 to 4 carbon atoms. The volatile alcohol may be selected from the group consisting of alkoxylated alcohols having a maximum of 8 carbon atoms, ethanol, propanol, isopropanol, and mixtures thereof. The volatile alcohol may be selected from the group consisting of isopropanol, $CH_3CH_2OCH_2CH_2OCH_2CH_2OH$, $CH_3OCH_2CH(CH_3)OCH_2CH(CH_3)OH$, $CH_3CH(OH)CH_2OCH_2CH(CH_3)OCH_3$, $CH_3CH_2OH$, and mixtures thereof. Miscibility in water can be useful in view of the advantages of using water as a solvent. Indeed, the solvent may be water and/or the majority of the solvent may be water. Since the present first aspect is "for providing a film on keratin fibers", the volatility of the alcohol can be chosen such that solvent and other non-film-forming compounds evaporate. A suitable product for providing the volatile alcohol can be Aquasolved Super from Firmenich.

The hair coloring composition may comprise a first anti-freeze agent and a further anti-freeze agent. The further anti-freeze agent may modify the effects of the first anti-freeze agent, for example, by altering any negative effects of the first anti-freeze agent. The hair coloring composition may comprise from 0.1% to 2%, or from 0.2% to 1% of a further anti-freeze agent by total weight of the composition. The further anti-freeze agent may be a surfactant, for example a non-ionic surfactant. The further anti-freeze agent may be a non-ionic surfactant having from 20 to 50 carbon atoms, or from 22 to 25 carbon atoms. The non-ionic surfactant may be an ether of a polyethylene glycol. The non-ionic surfactant may be a $C_{5-32}$ alkyl alcohol ether of a polyethylene glycol. The non-ionic surfactant may be a $C_{10-25}$ alkyl alcohol ether of a polyethylene glycol. Said polyethylene glycol may conform to the formula $H(OCH_2CH_2)_nOH$ wherein n is an integer from 1 to 20, or from 5 to 12. The non-ionic surfactant may be an ether of triglycerin, hexaglycerin, PPG-4, PPG-6, PEG-5, PEG-6, PEG-8, PEG-12, PEG-14, PEG-18, or PEG-20. A suitable non-ionic surfactant may be Tergitol™ 15-S-9 from Dow, which has the INCI name C11-15 Pareth-9 and may be the polyethylene glycol ether of a mixture of synthetic $C_{11-15}$ fatty alcohols with an average of 9 moles of ethylene oxide. Another suitable non-ionic surfactant may be trideceth-12.

Preservative

The hair coloring composition may comprise at least one preservative and/or a mixture of preservatives. The preservative and/or mixture of preservatives may be active against gram negative bacteria, *Staphylococcus aureus* and *Candida albicans.* The hair coloring composition may comprise 2-phenoxyethanol and/or phenylmethanol. The hair coloring composition may comprise 2-phenoxyethanol. The composition may comprise from 0.01% to 5% of a preservative, or from 0.1% to 2%, or from 0.5% to 1.5% of a preservative by total weight of the composition. The preservative may be selected from the group consisting of benzyl alcohol, phenoxyethanol, and mixtures thereof. The hair coloring composition may comprise at least one preservative; and wherein the preservative may be selected from the group consisting of benzyl alcohol and phenoxyethanol; or wherein the preservative may be a mixture of benzyl alcohol and phenoxyethanol.

The hair coloring composition may be substantially free of esters of parahydroxybenzoic acid. Esters of parahydroxybenzoic acid are commonly known as parabens.

The hair coloring composition may be substantially free of isothiazolinone compounds. The hair coloring composition may be substantially free of benzoate compounds. The composition may be substantially free of 1,3-bis(hydroxymethyl)-5,5-dimethylimidazolidine-2,4-dione.

Rheology

The hair coloring composition may have a viscosity of from 30 mPa·s to 1000 mPa·s. The viscosity is measured at 23°C using a HAAKE Rotation Viscometer VT 550 with cooling/heating vessel and sensor systems according to DIN 53019 at a shear rate of 12.9 $s^{-1}$. The hair coloring composition may have a viscosity of from 100 mPa·s to 500 mPa·s, or from 150 mPa·s to 450 mPa·s, or from 200 mPa·s to 400 mPa·s, or from 250 mPa·s to 350 mPa·s. The hair coloring composition may have a viscosity of from 100 mPa·s to 200 mPa·s. The viscosity range is useful in view of helping to prevent the hair coloring composition from dripping onto clothes and/or surrounding material. Furthermore, when the viscosity is too high, the hair coloring composition cannot easily be mixed.

The hair coloring composition may have a tangent delta of less than 2 at an angular frequency of 1 Hz at 23°C and at 1% strain. Tangent delta (also known as: tan delta, tan [δ], loss tangent, loss factor) is the ratio of viscous modulus (G") to elastic modulus (G') and is a quantifier of the presence and extent of elasticity in a fluid. How to calculate the tan delta is shown below:

**G' = Storage Modulus**
**G" = Loss Modulus**
**tan** δ = **Loss Factor**

$$\frac{G''}{G'} = \tan\delta$$

$$|G^*|(w) = G'(w) + iG''(w) = \sqrt{G'^2 + G''^2}$$

The storage modulus and tangent delta may be measured using a TA-Instruments AR2000ex rheometer. In the experiment, all the components of the hair coloring composition are mixed together, and then homogenised using an IKA KS 501 digital at 160 rpm and then loaded it onto the rheometer. The rheometer is used with the following specifications/conditions: upper geometry, 60 mm steel; lower geometry, Peltier Element; temperature, 23 °C; Pre-shear, 5 1/s, 1 min; equilibration, 2 min; normal force, off; gap: 650 μm. An amplitude sweep (strain sweep) was carried out with a strain of between 0.05% and 50%, with log data sampling (10 points/decade) and at a frequency of 1 Hz (6.283 rad/s).

The hair coloring composition may have a tangent delta of from 0.6 to 2 at an angular frequency of 1 Hz at 23°C and at 1% strain. The hair coloring composition may have a tangent delta of from 0.6 to 1.5, or from 0.6 to 1.0 at an angular frequency of 1 Hz at 23°C and at 1% strain.

Use

The hair coloring composition as set out hereinbefore can be used for highlighting already dyed hair fibers. Indeed, dyed hair may already comprise different shades of color. The hair color composition can help to improve the glowing of some shades due to the hair coloring agent and the film-forming aminosilicone polymer. Hence, the coating of relatively light dyed hair fibers with the hair coloring composition of the present invention can help to improve the

glowing effect of the relatively light dyed hair fibers with the hair coloring agent, e.g. a copper pigment, deposited on the hair fibers.

Method

Another aspect relates to a method for providing a film onto keratin fibers, the method comprising applying the hair coloring composition as set out herein above onto keratin fibers and allowing the keratin fibers to dry or drying them. The method may comprise applying the hair coloring composition as set out herein above onto keratin fibers and then drying them with a device selected from the group consisting of blow dryer, heated irons, heated hood, and combinations thereof. The method may preferably comprise applying the hair coloring composition as set out herein above onto keratin fibers with an applicator such as a sponge head.

The drying may be carried out using a hair dryer or a drying hood. The drying may be carried out at a temperature from 28°C to 40°C. The temperature may be useful in that it assists in the evaporation of solvent and other volatile compounds and thus allows excellent film formation. The method may comprise drying the keratin fibers with a device selected from the group consisting of blow dryer, heated irons, heated hood, and combinations thereof.

The method may comprise applying a first hair coloring composition onto keratin fibers and allowing the keratin fibers to dry or drying them; and subsequently, applying a second hair coloring composition onto keratin fibers and allowing the keratin fibers to dry or drying them; wherein the first hair coloring composition may comprise a first pigment exhibiting a first color and the hair coloring second composition may comprise a second pigment exhibiting a second color. The first color may be white or black. The second color may be neither white nor black. The first pigment may comprise titanium dioxide or carbon.

The method may not encompass or include bleaching the hair.

Kit

A kit is provided and comprises: the hair coloring composition as set out herein above; and an applicator. The kit may preferably consist of a packaging comprising the hair coloring composition as set out herein above and an applicator comprising a sponge head; and wherein the hair coloring composition may comprise one or more pigments and/or one or more colored materials. The kit may comprise a multi-compartment package comprising a first compartment and a second compartment; wherein the first compartment may comprise the hair coloring composition as set out herein above and the second compartment may comprise an applicator.

The kit may comprise a first hair coloring composition comprising a first pigment and a second hair coloring composition comprising a second pigment; wherein the first pigment and the second pigment may exhibit different colors.

The kit may also comprise an instructional material for use of the hair color composition. The instructional material can contain instructions how to use the hair color composition with the applicator. Also, the instructional material can contain further instructions how to prepare the hair coloring composition, and how to use the different compositions of the kit.

When the hair color composition comprises as a hair coloring agent an oxidation dye, the dye composition and the developer composition may be packaged in separate sealed containers in the kit. The dye composition may be packaged in a flexible tube packaging composed of metal, plastics or a combination thereof. The developer composition may be packaged in a squeezable container. The squeezable container may have at least 50% headspace. The squeezable container may have a headspace being at least the volume of the hair coloring composition. The developer composition may be packaged in a plastic container according to claim 1 of European Patent Application EP 2 801 281 A1, wherein the plastic container has two symmetrical collapsible side panels and a non-collapsible squeezable back panel; wherein the ratio of the average thicknesses between front and/or back panels and the side panels is at least 2:1 (See EP 2 801 281 A1 from paragraphs [0025] to [0044] as well as the Figures). The plastic container has the advantage that it is resistant to random, uncontrolled deformation under a substantial pressure differential between the environment and inside the container, yet having an affordable cost of manufacture and/or being appealing to the consumer.

EXAMPLES

The following examples further describe and demonstrate embodiments within the scope of the present invention. The examples are given solely for the purpose of illustration, and are not to be construed as limitations of the present invention since many variations thereof are possible without departing from its scope.

Hair coloring compositions:

| Ingredient / Example | 1 | 2 | 3 | 4 | 5 | 6 | 7 | 8 | 9 | 10 | 11 | 12 |
|---|---|---|---|---|---|---|---|---|---|---|---|---|
| Silsoft® CLX-E | 10 | 8 | 12 | 15 | 14 | 18 | 15 | 14 | 12 | 15 | 15 | 15 |
| Salcare® SC 96 [2] | 0.6 | 0.75 | 0.75 | 0.75 | - | - | 0.75 | - | 0.75 | 0.6 | 0.5 | 0.75 |
| 2-hydroxyethyl cellulose [3] | - | - | - | - | 0.2 | - | - | - | - | - | - | - |
| Xanthan gum [4] | - | - | - | - | - | - | - | 0.3 | - | - | - | - |
| PEG-45M [5] | 0.2 | 0.3 | - | - | 0.1 | - | 0.2 | - | - | 0.2 | 0.2 | 0.2 |
| PEG-23M [6] | - | - | 0.2 | - | - | - | - | - | 0.2 | - | - | - |
| PEG-90M [7] | - | - | - | 0.1 | - | - | - | - | - | - | - | - |
| PEG-7M [8] | - | - | - | - | 0.3 | - | - | - | - | - | - | - |
| PEG-180M [9] | - | - | - | - | - | - | - | 0.1 | - | - | - | - |
| Tergitol [$] | - | 0.5 | - | 0.5 | - | - | 0.5 | - | 0.5 | 0.5 | 0.5 | 0.5 |
| Pigment [§] | 5.0 | 5.0 | 5.0 | - | 5.0 | 5.0 | 5.0 | 5.0 | 5.0 | 5.0 | 5.0 | 5.0 |
| Aquasolved [#] | - | - | - | 2.5 | 2.5 | - | - | 2.3 | - | - | - | - |
| Ethanol | 1.0 | 1.5 | 2.0 | - | - | 1.0 | 1.5 | 2.0 | - | 1.0 | 1.0 | 1.0 |
| Preservative* | 1.0 | 2.0 | 1.5 | 0.5 | 1.0 | - | - | - | 1.0 | 1.0 | 1.0 | 1.0 |
| Perfume | - | 0.1 | 0.07 | 0.1 | - | - | - | 0.2 | 0.2 | 0.1 | 0.1 | 0.1 |

| | | | | | | | | | | | | |
|---|---|---|---|---|---|---|---|---|---|---|---|---|
| Deionised water | QSP | QSP | QSP | QSP | QSP | QSP | QSP | QSP | QSP | QSP | QSP | QSP |

KEY:

[1] = from Momentive Performance Materials, Albany, New York, (US). Silsoft® CLX-E has as the INCI name "dipropylene glycol and polysilicone-19". Polysilicone-29 is a complex silicone polymer formed by the reaction between a glycidoxypropyl-terminated dimethyl siloxane polymer, PEG-13 diglycidyl ether, diethylaminopropylamine, and aminopropyltriisopropoxysilane;

[2] = Salcare® SC 96 from BASF, which has the INCI name: Polyquaternium-37 (and) Propylene Glycol Dicaprate Dicaprylate (and) PPG-1 Trideceth-6;

[3] = Cellosize™ HEC QP 4400 from Dow;

[4] = Keltrol® from Kelco or Natrosol® 250 HHR from Herkules;

[5] = POLYOX WSR N-60K from Dow has formula $H(OCH_2CH_2)_nOH$ wherein n is an integer and where n has an average value of 45,000 and the weight average molecular weight is 2,000,000 Dalton; [6] = POLYOX WSR N-12K from Dow has the formula $H(OCH_2CH_2)_nOH$ wherein n is an integer with an average value of 23,000 and the weight average molecular weight is 1,000,000 Dalton;

[7] = POLYOX WSR-301 from Dow has the formula $H(OCH_2CH_2)_nOH$ wherein n is an integer with an average value of 90,000 and the weight average molecular weight is 4,000,000 Dalton;

[8] = POLYOX N-750 from Dow has the formula $H(OCH_2CH_2)_nOH$ wherein n is an integer with an average value of 7,000 and the weight average molecular weight is 300,000 Dalton;

[9] = POLYOX WSR-308 from Dow, as formula $H(OCH_2CH_2)_nOH$ wherein n is an integer and where n has an average value of 180,000 and has a weight average molecular weight of 8,000,000 Dalton;

[$] = C11-15 Pareth-9 and is the polyethylene glycol ether of a mixture of synthetic C11-15 fatty alcohols with an average of 9 moles of ethylene oxide.

* = 2-phenoxyethanol and/or phenylmethanol;

[#] = from Firmenich;

[§] = pigment selected from: mica and/or iron oxides; Colorona Bronze Fine; SynCrystal Almond; Xirona Le Rouge; DUOCROME RV 524C; SynCrystal Jade; Colorona Precious Gold; Ronastar Red; Syncrystal Sapphire; Impact Silver Rutile; KTZ Misterioso Pewter; copper pigment from Colorona®; and combinations thereof.

EXPERIMENTAL

**Color and Hair Performance**

The following compositions were prepared (all amounts are in wt%). A composition A comprises as a film-forming polymer Silsoft® CLX-E while a comparative composition B does not comprise any film-forming polymer. The comparative composition B is not within the scope of the present invention.

| Composition | Water | Pigment§ | Z (% wt.) | Total |
|---|---|---|---|---|
| A | QSP | 5 | 17% (2.55% active) | 100 |
| B | QSP | 5 | 0 | 100 |
| KEY: Z = from Momentive Performance Materials, Albany, New York, (US). Silsof® CLX-E has as the INCI name "dipropylene glycol and polysilicone-19". Polysilicone-29 is a complex silicone polymer formed by the reaction between a glycidoxypropyl-terminated dimethyl siloxane polymer, PEG-13 diglycidyl ether, diethylaminopropylamine, and aminopropyltriisopropoxysilane; § = copper pigment from Colorona®. | | | | |

Hair strands

Caucasian Hair color 4/0 Euro-Natural-Hair
Hair strands having a width of 1.5 cm and a length of 10 cm.
Available from Kerling International Haarfabrik

Application of each of the compositions A or B on hair strands

1.0 g of a composition A or a comparative composition B was applied on 1.0 g of hair strands. The respective composition A or B was distributed into the hair strands with the help of a sponge head. The hair strands were flipped. The respective composition A or B was further distributed until all hair fibers of the hair strands were completely and evenly colored.

Each treated hair strand was blow dried manually with a conventional blow dryer for 2 minutes. After each 30 seconds, the treated hair strands were combed from the top to the bottom of the hair strands.

Visual assessment of the color effect/intensity:

The hair strands treated with the composition A or B were compared to a reference, i.e. which was a non-treated hair strand, under a D65 light box. The assessment was rated as follows:

| -3 | -2 | -1 | 0 | 1 | 2 | 3 |
|---|---|---|---|---|---|---|
| Obvious worst | Noticeable worst | Slightly worst | Equal | Slightly better | Noticeable better | Obvious better |

Hair strands treated with the composition A exhibited a noticeable better effect on hair than the reference.

Hair strands treated with the comparative composition B exhibited a slight worst effect on hair than the reference.

When the composition comprises a film-forming polymer according to the present invention, the color effect on hair is better noticeable than when the composition does not comprise any film-forming polymer.

Hair Feel

The hair strands treated with the respective composition A or B were pulled through thumb and index fingers. The treated hair strands were assessed versus an untreated hair strand. The hair strands treated with the composition A showed a noticeable coated effect. However, the hair strands treated with the comparative composition B only showed a slight coated effect.

Water dipping Test

The hair strands treated with the respective composition A or B were dipped for 15 times for 15 seconds in 200

mL of distillate water. A picture was taken after the pigments have sedimented. It has been observed that the hair strands treated with the composition A lost few pigments. However, the hair strands treated with the comparative composition B lost much more pigments versus the hair strands treated with the composition A.

Conclusion

When the hair strands are treated with the composition A which comprises a film-forming polymer according to the present invention, versus the comparative composition B, the treated hair strands exhibited an improved colored performance, with an increased pigment adhesion on hair.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

**1.** A hair coloring composition for providing a film on keratin fibers, the composition comprising in a cosmetically acceptable carrier:

(a) a film-forming aminosilicone polymer, wherein the film-forming aminosilicone polymer is the product of the reaction of:

a1) a siloxane comprising at least two oxirane or oxetane groups having the formula:

$$M_fM^E_hM^{PE}_iM^H_jD_kD^E_lD^{PE}_mD^H_nT_oT^E_pT^{PE}_qT^H_rQ_s$$

With

$M = R^9R^{10}R^{11}SiO_{1/2}$;
$M^H = R^{12}R^{13}HSiO_{1/2}$;
$M^{PE} = R^{12}R^{13}(-CH_2CH(R^{14})(R^{15})_tO(R^{16})_u(C_2H_4O)_v(C_3H_6O)_w(C_4H_8O)_xR^{17})SiO_{1/2}$;
$M^E = R^{12}R^{13}(R^E)SiO_{1/2}$;
$D = R^{18}R^{19}SiO_{2/2}$; and
$D^H = R^{20}HSiO_{2/2}$;
$D^{PE} = R^{20}(-CH_2CH(R^{14})(R^{15})_tO(R^{16})_u(C_2H_4O)_v(C_3H_6O)_w(C_4H_8O)_xR^{17})SiO_{2/2}$;
$D^E = R^{20}R^ESiO_{2/2}$;
$T = R^{21}SiO_{3/2}$;
$T^H = HSiO_{3/2}$;
$T^{PE} = (-CH_2CH(R^{14})(R^{15})_tO(R^{16})_u(C_2H_4O)_v(C_3H_5O)_w(C_4H_8O)_xR^{17})SiO_{3/2}$;
$T^E = R^ESiO_{3/2}$; and
$Q = SiO_{4/2}$;
Where
$R^9$, $R^{10}$, $R^{11}$, $R^{12}$, $R^{13}$, $R^{18}$, $R^{19}$, $R^{20}$, and $R^{21}$ are each independently selected from the group of monovalent hydrocarbon radicals having from 1 to 60 carbon atoms;
$R^{14}$ is H or a 1 to 6 carbon atom alkyl group;
$R^{15}$ is a divalent alkyl radical of 1 to 6 carbons;
$R^{16}$ is selected from the group of divalent radicals consisting of $-C_2H_4O-$, $-C_3H_6O-$, and $-C_4H_8O-$;
$R^{17}$ is selected from the group consisting of H, monofunctional hydrocarbon radicals of 1 to 6 carbons, and acetyl;
$R^E$ is independently a monovalent hydrocarbon radical containing one or more oxirane or oxetane moieties having from 1 to 60 carbon atoms;
the subscript f is 0 or positive subject to the limitation that when the subscript f is 0, h must be positive;
the subscript h is 0 or positive subject to the limitations that when h is 0, the subscript f is positive, and that the sum of the subscripts h, 1 and p is positive;
the subscript k is zero or positive and has a value ranging from 0 to 1000;
the subscript 1 is 0 or positive and has a value ranging from 0 to 400 subject to the limitation that the

sum of the subscripts h, 1 and p is positive;
the subscript o is 0 or positive and has a value ranging from 0 to 50;
the subscript p is 0 or positive and has a value ranging from 0 to 30 subject to the limitation that the sum of the subscripts h, 1 and p is positive;
the subscript s is 0 or positive and has a value ranging from 0 to 20;
the subscript i is 0 or positive and has a value ranging from 0 to 20;
the subscript m is 0 or positive and has a value ranging from 0 to 200;
the subscript q is 0 or positive and has a value ranging from 0 to 30;
the subscript j is 0 or positive and has a value ranging from 0 to 2;
the subscript n is 0 or positive and has a value ranging from 0 to 20;
the subscript r is 0 or positive and has a value ranging from 0 to 30;
the subscript t is 0 or 1;
the subscript u is 0 or 1;
the subscript v is 0 or positive and has a value ranging from 0 to 100 subject to the limitation that (v+w+x) > 0;
the subscript w is 0 or positive and has a value ranging from 0 to 100 subject to the limitation that (v+w+x) > 0;
the subscript x is 0 or positive and has a value ranging from 0 to 100 subject to the limitation that (v + w + x) > 0; and

a2) an aminosilane having the formula:

$$N(H)(R^1)R^2Si(OR^3)_{3-a-b-c}(OR^4)_a(R^5Si(OR^6)_d(R^7)_e)_bR^8_c$$

with

$R^1$ is chosen from the group consisting of H or a monovalent hydrocarbon radical containing 1 to 20 carbon atoms;
$R^2$ is selected from a group consisting of a divalent linear or branched hydrocarbon radical consisting of 1 to 60 carbons;
$R^4$ is a hydrocarbon radical that contains 3 to 200 carbon atoms;
$R^5$ is selected from a group consisting of oxygen or a divalent linear or branched hydrocarbon radical consisting of 1 to 60 carbons;
$R^3$, $R^6$, $R^7$, and $R^8$ and are each independently selected from the group of monovalent linear or branched hydrocarbon radicals having from 1 to 200 carbon atoms;
the subscript b is 0 or a positive number and has a value ranging from 0 to 3;
the subscript a is a positive number less than 3;
the subscripts b and c are 0 or positive and have a value ranging from 0 to 3 subject to the limitation that $(a+b+c) \leq 3$;
the subscripts d and e are 0 or positive and have a value ranging from 0 to 3 subject to the limitation that (d+e) = 3; and

(b) a hair coloring agent.

**2.** The hair coloring composition of claim 1, wherein the film-forming aminosilicone polymer is the product of the reaction of the siloxane (a1), the aminosilane (a2), and a compound (I) having the formula:

(I) $$R^{29}(R^{30})_\kappa Si(OR^{31})_{3-\eta-\theta}(R^{32})_\eta(OR^{33})_\theta$$

where

$R^{29}$ is a monovalent hydrocarbon radical containing one or more oxirane or oxetane moieties having from 3 to 12 carbon atoms;
$R^{30}$ is a divalent hydrocarbon radical consisting of 1 to 60 carbons and the subscript $\kappa$ has a value of 0 or 1;
$R^{31}$ and $R^{32}$ are independently selected from the group of monovalent linear or branched hydrocarbon radicals having from 1 to 60 carbon atoms;
the subscript $\eta$ is zero or positive and has a value ranging from 0 to 3;
the subscript $\theta$ is > 0 and $\leq 3$, subject to the limitation that $3-\eta-\theta \geq 0$;

$R^{33}$ is a hydrocarbon radical that contains 3 to 200 carbon atoms.

**3.** The hair coloring composition of claim 2, wherein:

$R^1$ has from 1 to 5 carbon atoms;
$R^2$ has from 2 to 8 carbon atoms;
$R^4$ has from 3 to 8 carbon atoms;
$R^3$, $R^6$, $R^7$, and $R^8$ each independently have from 1 to 15 carbon atoms;
the subscript a ranges from 2 to 3;
the subscript b ranges from 0 to 15;
the subscript c ranges from 0 to 2;
$R^9$, $R^{10}$, $R^{11}$, $R^{12}$, $R^{13}$, $R^{18}$, $R^{19}$, $R^{20}$, and $R^{21}$ each independently have from 1 to 3 carbon atoms;
the subscript k ranges from 5 to 250;
the subscripts v, w, and x each independently range from 0 to 35;
$R^{31}$ and $R^{32}$ each independently have from 1 to 8 carbon atoms, and
$R^{33}$ has from 3 to 50 carbon atoms.

**4.** The hair coloring composition of claim 2, wherein:

$R^1$ is H;
$R^2$ has from 2 to 5 carbon atoms;
$R^4$ has from 3 to 5 carbon atoms;
$R^3$, $R^6$, $R^7$, and $R^8$ each independently have from 2 to 8 carbon atoms;
the subscript a ranges from 0 or 1;
the subscript b is 3;
the subscript c is 0 or 1;
$R^9$, $R^{10}$, $R^{11}$, $R^{12}$, $R^{13}$, $R^{18}$, $R^{19}$, $R^{20}$, and $R^{21}$ are each independently methyl;
the subscript k ranges from 5 to 150;
the subscripts v, w, and x each independently range from 0 to 25;
$R^{31}$ and $R^{32}$ each independently have from 1 to 4 carbon atoms; and
$R^{33}$ has from 3 to 10 carbon atoms.

**5.** The hair coloring composition of claim 1, wherein:

$R^1$ is H;
$R^2$ has from 2 to 5 carbon atoms;
$R^3$ has from 3 to 5 carbon atoms;
$R^4$ has from 3 to 5 carbon atoms;
$R^3$, $R^6$, $R^7$, and $R^8$ each independently have from 2 to 8 carbon atoms;
the subscript a is 1;
the subscript b is 0;
the subscript c is 0;
$R^9$, $R^{10}$, $R^{11}$, $R^{12}$, $R^{13}$, $R^{18}$, $R^{19}$, $R^{20}$, and $R^{21}$ are each independently methyl;
the subscript k ranges from 5 to 150;
the subscripts v, w, and x each independently range from 0 to 25;
the subscript n is 0.

**6.** The hair coloring composition of claim 1, wherein:
the siloxane is an epoxy encapped polysiloxane with the average structure:

$$CH_2(O)CHCH_2O(CH_2)_\lambda Si(CH_3)_2O[Si(CH_3)_2O]_\kappa Si(CH_3)_2CH_2CH_2CH_2OCH_2CH(O)CH_2$$

with the subscript k ranges from 5 to 250, from 5 to 150, preferably from 50 to 100, and the subscript $\lambda$ is 2 or 3; and the aminosilane has the formula $H_2NR^2Si(OR^3)_2(OR^4)$ with
$R^2$ has from 2 to 5 carbon atoms;
$R^3$ has from 3 to 5 carbon atoms; and
$R^4$ has from 3 to 5 carbon atoms.

**7.** The hair coloring composition of claim 6, wherein the film-forming aminosilicone polymer is the product of the reaction of the siloxane (a1), the aminosilane (a2), and an encapped polyether with the average structure $CH_2(O)CHCH_2O(CH_2CH_2O)_{\mu}CH_2CH(O)CH_2$ with the subscript β ranges from 10 to 20, or an encapped polyether with the average structure $CH_2(O)CHCH_2O(CH_2CH_2O)_{\mu}(CH_2CHCH_3O)_{\nu}CH_2CH(O)CH_2$ with the subscript μ ranges from 1 to 20 and with the subscript v ranges from 1 to 20.

**8.** The hair coloring composition of claim 7, wherein the film-forming aminosilicone polymer is the product of the reaction of the siloxane (a1) and the aminosilane (a2) in the presence of a primary or a secondary amine, preferably a primary amine selected from the group consisting of, methylamine, ethylamine, propylamine, ethanol amine, isopropylamine, butylamine, isobutylamine, hexylamine, dodecylamine, oleylamine, aniline aminopropyltrimethylsilane, aminopropyltriethylsilane, aminomorpholine, 3-(diethylamino)propylamine, benzylamine, napthylamine 3-amino-9-ethylcarbazole, 1-aminoheptaphlorohexane, 2,2,3,3,4,4,5,5,6,6,7,7,8,8,8-pentadecafluoro-1-octanamine, and mixtures thereof or a secondary amine selected from the group consisting of methylethylamine, methylhexylamine, methyloctadecylamine, diethanolamine, dibenzylamine, dihexylamine, dicyclohexylamine, piperidine, pyrrolidine, phthalimide, and mixtures thereof.

**9.** The hair coloring composition of any preceding claim, wherein the hair coloring agent comprises one or more pigments, preferably wherein the one or more pigments have a $D_{50}$ particle diameter of from 5 micron to 60 micron.

**10.** The hair coloring composition of any preceding claim, wherein the hair coloring agent comprises one or more colored materials, preferably wherein the one or more colored materials are selected from the group consisting of: colored fibers, colored beads, colored particles such as nano-particles, colored polymers comprising covalently attached dyes, particles having diffraction properties, and combinations thereof.

**11.** The hair coloring composition of any one of the claims 1-8, wherein the hair coloring agent comprises a dye composition and optionally a developer composition wherein the developer composition comprises one or more oxidizing agents; and wherein the dye composition comprises one or more oxidative dye precursors comprising one or more couplers and one or more primary intermediates or wherein the dye composition consists essentially of one or more direct dyes.

**12.** The hair coloring composition of claim 11, wherein the one or more primary intermediates are selected from the group consisting of toluene-2,5-diamine, p-phenylenediamine, N-phenyl-p-phenylenediamine, N,N-bis(2-hydroxyethyl)-p-phenylenediamine, 2-hydroxyethyl-p-phenylenediamine, hydroxypropyl-bis-(N-hydroxyethyl-p-phenylenediamine), 2-methoxymethyl-p-phenylenediamine, 2-(1,2-dihydroxyethyl)-p-phenylenediamine, 2,2'-(2-(4-aminophenylamino)ethylazanediyl)diethanol, 2-(2,5-diamino-4-methoxyphenyl)propane-1,3-diol, 2-(7-amino-2H-benzo[b][1,4]oxazin-4(3H)-yl)ethanol, 2-chloro-p-phenylenediamine, p-aminophenol, p-(methylamino)phenol, 4-amino-m-cresol, 6-amino-m-cresol, 5-ethyl-o-aminophenol, 2-methoxy-p-phenylenediamine, 2,2'-methylenebis-4-aminophenol, 2,4,5,6-tetraminopyrimidine, 2,5,6-triamino-4-pyrimidinol, 1-hydroxyethyl-4,5-diaminopyrazole sulfate, 4,5-diamino-1-methylpyrazole, 4,5-diamino-1-ethylpyrazole, 4,5-diamino-1-isopropylpyrazole, 4,5-diamino-1-butylpyrazole, 4,5-diamino-1-pentylpyrazole, 4,5-diamino-1-benzylpyrazole, 2,3-diamino-6,7-dihydropyrazolo[1,2-a]pyrazol-1(5H)-one dimethosulfonate, 4,5-diamino-1-hexylpyrazole, 4,5-diamino-1-heptylpyrazole, methoxymethyl-1,4-diaminobenzene, N,N-bis(2-hydroxyethyl)-N-(4-aminophenyl)-1 ,2-diaminothane, 2-[(3-aminopyrazolo[1,5 - a]pyridin-2-yl)oxy]ethanol hydrochloride, salts thereof and mixtures thereof.

**13.** Use of the hair coloring composition according to any of claims 1 to 12 for highlighting already dyed hair fibers.

**14.** A method for providing a film onto keratin fibers, the method comprising applying the hair coloring composition according to any of claims 1 to 12 onto keratin fibers and allowing the keratin fibers to dry or drying them.

**15.** A kit comprising:

- the hair coloring composition according to any of claims 1 to 12; and
- an applicator.

**Patentansprüche**

**1.** Haarfärbezusammensetzung zum Versehen von Keratinfasern mit einem Film, wobei die Zusammensetzung in

einem kosmetisch akzeptablen Träger umfasst:

(a) ein filmbildendes Aminosiliconpolymer, wobei das filmbildende Aminosiliconpolymer das Produkt der Reaktion ist von:

a1) einem Siloxan, umfassend mindestens zwei Oxiran- oder Oxetangruppen mit der Formel:

$$M_fM^E_hM^{PE}_iM^H_jD_kD^E_lD^{PE}_mD^H_nT_oT^E_pT^{PE}_qT^H_rQ_s,$$

wobei

$M = R^9R^{10}R^{11}SiO_{1/2}$;
$M^H = R^{12}R^{13}HSiO_{1/2}$;
$M^{PE} = R^{12}R^{13}(-CH_2CH(R^{14})(R^{15})_tO(R^{16})_u(C_2H_4O)_v(C_3H_6O)_w(C_4H_8O)_xR^{17})SiO_{1/2}$;
$M^E = R^{12}R^{13}(R^E)SiO_{1/2}$;
$D = R^{18}R^{19}SiO_{2/2}$; und
$D^H = R^2HSiO_{2/2}$;
$D^{PE} = R^{20}(-CH_2CH(R^{14})(R^{15})_tO(R^{16})_u(C_2H_4O)_v(C_3H_6O)_w(C_4H_8O)_xR^{17})SiO_{2/2}$;
$D^E = R^{20}R^ESiO_{2/2}$;
$T = R^{21}SiO_{3/2}$;
$T^H = HSiO_{3/2}$;
$T^{PE} = (-CH_2CH(R^{14})(R^{15})_tO(R^{16})_u(C_2H_4O)_v(C_3H_5O)_w(C_4H_8O)_xR^{17})SiO_{3/2}$;
$T^E = R^ESiO_{3/2}$; und
$Q = SiO_{4/2}$;
wobei
$R^9$, $R^{10}$, $R^{11}$, $R^{12}$, $R^{13}$, $R^{18}$, $R^{19}$, $R^{20}$ und $R^{21}$ jeweils unabhängig aus der Gruppe von einwertigen Kohlenwasserstoffresten mit 1 bis 60 Kohlenstoffatomen ausgewählt sind;
$R^{14}$ H oder eine Alkylgruppe von 1 bis 6 Kohlenstoffatomen ist;
$R^{15}$ ein zweiwertiger Alkylrest mit 1 bis 6 Kohlenstoffatomen ist;
$R^{16}$ aus der Gruppe von zweiwertigen Resten ausgewählt ist, die aus $-C_2H_4O-$, $-C_3H_6O-$ und $-C_4H_8O-$ besteht;
$R^{17}$ aus der Gruppe ausgewählt ist, die aus H, monofunktionellen Kohlenwasserstoffresten mit 1 bis 6 Kohlenstoffen und Acetyl besteht;
$R^E$ unabhängig ein einwertiger Kohlenwasserstoffrest ist, der eine oder mehrere Oxiran- oder Oxetan-Molekülteile mit 1 bis 60 Kohlenstoffatomen enthält;
das Subskript f 0 oder positiv ist, mit der Einschränkung, dass, wenn das Subskript f 0 ist, h positiv sein muss;
das Subskript h 0 oder positiv ist, mit den Einschränkungen, dass, wenn h 0 ist, das Subskript f positiv ist und dass die Summe der Subskripte h, l und p positiv ist;
das Subskript k null oder positiv ist und einen Wert im Bereich von 0 bis 1.000 aufweist;
das Subskript 1 0 oder positiv ist und einen Wert im Bereich von 0 bis 400 aufweist, mit der Einschränkung, dass die Summe der Subskripte h, 1 und p positiv ist;
das Subskript o 0 oder positiv ist und einen Wert im Bereich von 0 bis 50 aufweist;
das Subskript p 0 oder positiv ist und einen Wert im Bereich von 0 bis 30 aufweist, mit der Einschränkung, dass die Summe der Subskripte h, 1 und p positiv ist;
das Subskript s 0 oder positiv ist und einen Wert im Bereich von 0 bis 20 aufweist;
das Subskript i 0 oder positiv ist und einen Wert im Bereich von 0 bis 20 aufweist;
das Subskript m 0 oder positiv ist und einen Wert im Bereich von 0 bis 200 aufweist;
das Subskript q 0 oder positiv ist und einen Wert im Bereich von 0 bis 30 aufweist;
das Subskript j 0 oder positiv ist und einen Wert im Bereich von 0 bis 2 aufweist;
das Subskript n 0 oder positiv ist und einen Wert im Bereich von 0 bis 20 aufweist;
das Subskript r 0 oder positiv ist und einen Wert im Bereich von 0 bis 30 aufweist;
das Subskript t 0 oder 1 ist;
das Subskript u 0 oder 1 ist;
das Subskript v 0 oder positiv ist und einen Wert im Bereich von 0 bis 100 aufweist, mit der Einschränkung, dass $(v + w + x) > 0$;
das Subskript w 0 oder positiv ist und einen Wert im Bereich von 0 bis 100 aufweist, mit der Einschränkung, dass $(v + w + x) > 0$;

das Subskript x 0 oder positiv ist und einen Wert im Bereich von 0 bis 100 aufweist, mit der Einschränkung, dass $(v + w + x) > 0$; und

a2) einem Aminosilan mit der Formel:

$$N(H)(R^1)R^2Si(OR^3)_{3-a-b-c}(OR^4)_a(R^5Si(OR^6)_d(R^7)_e)_bR^8_c,$$

wobei

$R^1$ aus der Gruppe ausgewählt ist, die aus H oder einem einwertigen Kohlenwasserstoffrest besteht, der 1 bis 20 Kohlenstoffatome enthält;
$R^2$ aus einer Gruppe ausgewählt ist, die aus einem zweiwertigen linearen oder verzweigten Kohlenwasserstoffrest besteht, der aus 1 bis 60 Kohlenstoffen besteht;
$R^4$ ein Kohlenwasserstoffrest ist, der 3 bis 200 Kohlenstoffatome enthält;
$R^5$ aus einer Gruppe ausgewählt ist, die aus Sauerstoff oder einem zweiwertigen linearen oder verzweigten Kohlenwasserstoffrest besteht, der aus 1 bis 60 Kohlenstoffen besteht;
$R^3$, $R^6$, $R^7$ und $R^8$ jeweils unabhängig aus der Gruppe von einwertigen linearen oder verzweigten Kohlenwasserstoffresten mit 1 bis 200 Kohlenstoffatomen ausgewählt sind;
das Subskript b 0 oder eine positive Zahl ist und einen Wert im Bereich von 0 bis 3 aufweist;
das Subskript a eine positive Zahl kleiner als 3 ist;
die Subskripte b und c 0 oder positiv sind und einen Wert im Bereich von 0 bis 3 aufweisen, mit der Einschränkung, dass $(a + b + c) \leq 3$;
die Subskripte d und e 0 oder positiv sind und einen Wert im Bereich von 0 bis 3 aufweisen, mit der Einschränkung, dass $(d + e) = 3$; und

(b) ein Haarfärbemittel.

**2.** Haarfärbezusammensetzung nach Anspruch 1, wobei das filmbildende Aminosiliconpolymer das Produkt der Reaktion des Siloxans (a1), des Aminosilans (a2) und einer Verbindung (I) mit der Formel ist:

$$\text{(I)} \qquad R^{29}(R^{30})_\kappa Si(OR^{31})_{3-\eta-\theta}(R^{32})_\eta(OR^{33})_\theta,$$

wobei

$R^{29}$ ein einwertiger Kohlenwasserstoffrest ist, der eine oder mehrere Oxiran- oder Oxetan-Molekülteile mit 3 bis 12 Kohlenstoffatomen enthält;
$R^{30}$ ein zweiwertiger Kohlenwasserstoffrest ist, der aus 1 bis 60 Kohlenstoffen besteht, und das Subskript $\kappa$ einen Wert von 0 oder 1 aufweist;
$R^{31}$ und $R^{32}$ unabhängig aus der Gruppe von einwertigen linearen oder verzweigten Kohlenwasserstoffresten mit 1 bis 60 Kohlenstoffatomen ausgewählt sind;
das Subskript $\eta$ null oder positiv ist und einen Wert im Bereich von 0 bis 3 aufweist;
das Subskript $\theta > 0$ und $\leq 3$ ist, mit der Einschränkung, dass $3-\eta-\theta \leq 0$;
$R^{33}$ ein Kohlenwasserstoffrest ist, der 3 bis 200 Kohlenstoffatome enthält.

**3.** Haarfärbezusammensetzung nach Anspruch 2, wobei:

$R^1$ 1 bis 5 Kohlenstoffatome aufweist;
$R^2$ 2 bis 8 Kohlenstoffatome aufweist;
$R^4$ 3 bis 8 Kohlenstoffatome aufweist;
$R^3$, $R^6$, $R^7$ und $R^8$ jeweils unabhängig 1 bis 15 Kohlenstoffatome aufweisen;
das Subskript a im Bereich von 2 bis 3 ist;
das Subskript b im Bereich von 0 bis 15 ist;
das Subskript c im Bereich von 0 bis 2 ist;
$R^9$, $R^{10}$, $R^{11}$, $R^{12}$, $R^{13}$, $R^{18}$, $R^{19}$, $R^{20}$ und $R^{21}$ jeweils unabhängig 1 bis 3 Kohlenstoffatome aufweisen;
das Subskript k im Bereich von 5 bis 250 ist;
die Subskripte v, w und x jeweils unabhängig im Bereich von 0 bis 35 sind;
$R^{31}$ und $R^{32}$ jeweils unabhängig 1 bis 8 Kohlenstoffatome aufweisen und
$R^{33}$ 3 bis 50 Kohlenstoffatome aufweist.

**4.** Haarfärbezusammensetzung nach Anspruch 2, wobei:

$R^1$ H ist;
$R^2$ 2 bis 5 Kohlenstoffatome aufweist;
$R^4$ 3 bis 5 Kohlenstoffatome aufweist;
$R^3$, $R^6$, $R^7$ und $R^8$ jeweils unabhängig 2 bis 8 Kohlenstoffatome aufweisen;
das Subskript a im Bereich von 0 oder 1 ist;
das Subskript b 3 ist;
das Subskript c 0 oder 1 ist;
$R^9$, $R^{10}$, $R^{11}$, $R^{12}$, $R^{13}$, $R^{18}$, $R^{19}$, $R^{20}$ und $R^{21}$ jeweils unabhängig Methyl sind;
das Subskript k im Bereich von 5 bis 150 ist;
die Subskripte v, w und x jeweils unabhängig im Bereich von 0 bis 25 sind;
$R^{31}$ und $R^{32}$ jeweils unabhängig 1 bis 4 Kohlenstoffatome aufweisen; und
$R^{33}$ 3 bis 10 Kohlenstoffatome aufweist.

**5.** Haarfärbezusammensetzung nach Anspruch 1, wobei:

$R^1$ H ist;
$R^2$ 2 bis 5 Kohlenstoffatome aufweist;
$R^3$ 3 bis 5 Kohlenstoffatome aufweist;
$R^4$ 3 bis 5 Kohlenstoffatome aufweist;
$R^3$, $R^6$, $R^7$ und $R^8$ jeweils unabhängig 2 bis 8 Kohlenstoffatome aufweisen;
das Subskript a 1 ist;
das Subskript b 0 ist;
das Subskript c 0 ist;
$R^9$, $R^{10}$, $R^{11}$, $R^{12}$, $R^{13}$, $R^{18}$, $R^{19}$, $R^{20}$ und $R^{21}$ jeweils unabhängig Methyl sind;
das Subskript k im Bereich von 5 bis 150 ist;
die Subskripte v, w und x jeweils unabhängig im Bereich von 0 bis 25 sind;
das Subskript n 0 ist.

**6.** Haarfärbezusammensetzung nach Anspruch 1, wobei:

das Siloxan ein epoxidverkapptes Polysiloxan mit der durchschnittlichen Struktur ist:

$$CH_2(O)CHCH_2O(CH_2)_\lambda Si(CH_3)_2O[Si(CH_3)_2O]_kSi(CH_3)_2CH_2CH_2CH_2OCH_2C\ H(O)CH_2,$$

wobei das Subskript k im Bereich von 5 bis 250, von 5 bis 150, bevorzugt von 50 bis 100, ist und das Subskript $\lambda$ 2 oder 3 ist; und

das Aminosilan die Formel $H_2NR^2Si(OR^3)_2(OR^4)$ aufweist, wobei
$R^2$ 2 bis 5 Kohlenstoffatome aufweist;
$R^3$ 3 bis 5 Kohlenstoffatome aufweist; und
$R^4$ 3 bis 5 Kohlenstoffatome aufweist.

**7.** Haarfärbezusammensetzung nach Anspruch 6, wobei das filmbildende Aminosiliconpolymer das Produkt der Reaktion des Siloxans (a1), des Aminosilans (a2) und eines verkappten Polyethers mit der durchschnittlichen Struktur $CH_2(O)CHCH_2O(CH_2CH_2O)_\mu CH_2CH(O)CH_2$ ist, wobei das Subskript $\beta$ im Bereich von 10 bis 20 ist, oder eines verkappten Polyethers mit der durchschnittlichen Struktur $CH_2(O)CHCH_2O(CH_2CH_2O)_\mu(CH_2CHCH_3O)_v CH_2CH(O)CH_2$ ist, wobei das Subskript $\mu$ im Bereich von 1 bis 20 ist und wobei das Subskript v im Bereich von 1 bis 20 ist.

**8.** Haarfärbezusammensetzung nach Anspruch 7, wobei das filmbildende Aminosiliconpolymer das Produkt der Reaktion des Siloxans (a1) und des Aminosilans (a2) in Gegenwart eines primären oder eines sekundären Amins, bevorzugt eines primären Amins, ist, das aus der Gruppe ausgewählt ist, die aus Methylamin, Ethylamin, Propylamin, Ethanolamin, Isopropylamin, Butylamin, Isobutylamin, Hexylamin, Dodecylamin, Oleylamin, Anilinaminopropyltrimethylsilan, Aminopropyltriethylsilan, Aminomorpholin, 3-(Diethylamino)propylamin, Benzylamin, Naphthylamin-3-amino-9-ethylcarbazol, 1-Aminoheptachlorhexan, 2,2,3,3,4,4,S,S,6,6,7,7,S,S,S-Pentadecafluor-1-octanamin und

Gemischen davon, oder eines sekundären Amins ist, das aus der Gruppe ausgewählt ist, die aus Methylethylamin, Methylhexylamin, Methyloctadecylamin, Diethanolamin, Dibenzylamin, Dihexylamin, Dicyclohexylamin, Piperidin, Pyrrolidin, Phthalimid und Gemischen davon besteht.

**9.** Haarfärbezusammensetzung nach einem vorhergehenden Anspruch, wobei das Haarfärbemittel ein oder mehrere Pigmente umfasst, wobei das eine oder die mehreren Pigmente bevorzugt einen $D_{50}$-Teilchendurchmesser von 5 Mikrometern bis 60 Mikrometern aufweisen.

**10.** Haarfärbezusammensetzung nach einem vorhergehenden Anspruch, wobei das Haarfärbemittel ein oder mehrere farbige Materialien umfasst, wobei das eine oder die mehreren farbigen Materialien bevorzugt aus der Gruppe ausgewählt sind, die besteht aus: farbigen Fasern, farbigen Kügelchen, farbigen Teilchen wie z. B. Nanoteilchen, farbigen Polymeren, umfassend kovalent befestigte Farbstoffe, Teilchen mit Beugungseigenschaften und Kombinationen davon.

**11.** Haarfärbezusammensetzung nach einem von Anspruch 1 bis 8, wobei das Haarfärbemittel eine Farbstoffzusammensetzung und wahlweise eine Entwicklerzusammensetzung umfasst, wobei die Entwicklerzusammensetzung ein oder mehrere Oxidationsmittel umfasst; und wobei die Farbstoffzusammensetzung einen oder mehrere oxidative Farbstoffvorläufer umfasst, umfassend einen oder mehrere Kuppler und ein oder mehrere primäre Zwischenprodukte, oder wobei die Farbstoffzusammensetzung im Wesentlichen aus einem oder mehreren Direktfarbstoffen besteht.

**12.** Haarfärbezusammensetzung nach Anspruch 11, wobei das eine oder die mehreren primären Zwischenprodukte aus der Gruppe ausgewählt sind, die aus Toluol-2,5-diamin, p-Phenylendiamin, N-Phenyl-p-phenylendiamin, N,N-Bis(2-hydroxyethyl)-p-phenylendiamin, 2-Hydroxyethyl-p-phenylendiamin, Hydroxypropyl-bis-(N-hydroxyethyl-p-phenylendiamin), 2-Methoxymethyl-p-phenylendiamin, 2-(1,2-Dihydroxyethyl)-p-phenylendiamin, 2,2'-(2-(4-Aminophenylamino)ethylazandiyl)diethanol, 2-(2,5-Diamino-4-methoxyphenyl)propan-1,3-diol, 2-(7-Amino-2H-benzo[b][1,4]oxazin-4(3H)-yl)ethanol, 2-Chlor-p-phenylendiamin, p-Aminophenol, p-(Methylamino)phenol, 4-Amino-m-cresol, 6-Amino-m-cresol, 5-Ethyl-o-aminophenol, 2-Methoxy-p-phenylendiamin, 2,2'-Methylenbis-4-aminophenol, 2,4,5,6-Tetraminopyrimidin, 2,5,6-Triamino-4-pyrimidinol, 1-Hydroxyethyl-4,5-diaminopyrazolsulfat, 4,5-Diamino-1-methylpyrazol, 4,5-Diamino-1-ethylpyrazol, 4,5-Diamino-1-isopropylpyrazol, 4,5-Diamino-1-butylpyrazol, 4,5-Diamino-1-pentylpyrazol, 4,5-Diamino-1-benzylpyrazol, 2,3-Diamino-6,7-dihydropyrazolo[1,2-a]pyrazol-1(5H)-on-dimethosulfonat, 4,5-Diamino-1-hexylpyrazol, 4,5-Diamino-1-heptylpyrazol, Methoxymethyl-1,4-diaminobenzol, N,N-Bis(2-hydroxyethyl)-N-(4-aminophenyl)-1,2-diaminothan, 2-[(3-Aminopyrazolo[1,5-a]pyridin-2-yl)oxy]ethanol-hydrochlorid, Salzen davon und Gemischen davon besteht.

**13.** Benutzung der Haarfärbezusammensetzung nach einem von Anspruch 1 bis 12 zum Hervorheben von bereits gefärbten Haarfasern.

**14.** Verfahren zum Versehen von Keratinfasern mit einem Film, wobei das Verfahren das Aufbringen der Haarfärbezusammensetzung nach einem von Anspruch 1 bis 12 auf Keratinfasern und das Trocknenlassen oder Trocknen der Keratinfasern umfasst.

**15.** Kit, umfassend:

- o die Haarfärbezusammensetzung nach einem von Anspruch 1 bis 12; und
- ◦ einen Applikator.

## Revendications

**1.** Composition de coloration capillaire permettant de déposer un film sur des fibres de kératine, la composition comprenant, dans un support cosmétiquement acceptable :

(a) un polymère d'aminosilicone filmogène, dans laquelle le polymère d'aminosilicone filmogène est le produit de la réaction de :

a1) un siloxane comprenant au moins deux groupes oxirane ou oxétane de formule :

$$M_fM^E_hM^{PE}_iM^H_jD_kD^E_lD^{PE}_mD^H_nT_oT^E_pT^{PE}_qT^H_rQ_s$$

avec

$M = R^9R^{10}R^{11}SiO_{1/2}$ ;
$M^H = R^{12}R^{13}HSiO_{1/2}$ ;
$M^{PE} = R^{12}R^{13} (-CH_2CH(R^{14})(R^{15})_tO(R^{16})_u(C_2H_4O)_v(C_3H_6O)_w(C_4H_8O)_xR^{17})SiO_{1/2}$ ;
$M^E = R^{12}R^{13}(R^E)SiO_{1/2}$ ;
$D = R^{18}R^{19}SiO_{2/2}$ ; et
$D^H = R^{20}HSiO_{2/2}$ ;
$D^{PE} = R^{20}(-CH_2CH(R^{14})(R^{15})_tO(R^{16})_u(C_2H_4O)_v(C_3H_6O)_w(C_4H_8O)_xR^{17})SiO_{2/2}$ ;
$D^E = R^{20}R^ESiO_{2/2}$ ;
$T = R^{21}SiO_{3/2}$ ;
$T^H = HSiO_{3/2}$ ;
$T^{PE} = (-CH_2CH(R^{14})(R^{15})_tO(R^{16})_u(C_2H_4O)_v(C_3H_5O)_w(C_4H_8O)_xR^{17})SiO_{3/2}$ ;
$T^E = R^ESiO_{3/2}$ ; et
$Q = SiO_{4/2}$ ;
Où
$R^9$, $R^{10}$, $R^{11}$, $R^{12}$, $R^{13}$, $R^{18}$, $R^{19}$, $R^{20}$ et $R^{21}$ sont chacun indépendamment choisis dans le groupe de radicaux hydrocarbonés monovalents ayant de 1 à 60 atomes de carbone ;
$R^{14}$ est H ou un groupe alkyle de 1 à 6 atomes de carbone ;
$R^{15}$ est un radical alkyle divalent de 1 à 6 atomes de carbone ;
$R^{16}$ est choisi dans le groupe de radicaux divalents constitué par $-C_2H_4O-$, $-C_3H_6O-$ et $-C_4H_8O-$ ;
$R^{17}$ est choisi dans le groupe constitué par H, des radicaux hydrocarbonés monofonctionnels de 1 à 6 atomes de carbone et l'acétyle ;
$R^E$ est indépendamment un radical hydrocarboné monovalent contenant une ou plusieurs fractions d'oxirane ou d'oxétane ayant de 1 à 60 atomes de carbone ;
l'indice f vaut 0 ou est positif sous réserve que lorsque l'indice f vaut 0, h doive être positif ;
l'indice h vaut 0 ou est positif sous réserve que lorsque l'indice h vaut 0, l'indice f soit positif et que la somme des indices h, l et p soit positive ;
l'indice k vaut zéro ou est positif et a une valeur dans la plage de 0 à 1000 ;
l'indice l vaut 0 ou est positif et a une valeur dans la plage de 0 à 400 sous réserve que la somme des indices h, l et p soit positive ;
l'indice o vaut 0 ou est positif et a une valeur dans la plage de 0 à 50 ;
l'indice p vaut 0 ou est positif et a une valeur dans la plage de 0 à 30 sous réserve que la somme des indices h, l et p soit positive ;
l'indice s vaut 0 ou est positif et a une valeur dans la plage de 0 à 20 ;
l'indice i vaut 0 ou est positif et a une valeur dans la plage de 0 à 20 ;
l'indice m vaut 0 ou est positif et a une valeur dans la plage de 0 à 200 ;
l'indice q vaut 0 ou est positif et a une valeur dans la plage de 0 à 30 ;
l'indice j vaut 0 ou est positif et a une valeur dans la plage de 0 à 2 ;
l'indice n vaut 0 ou est positif et a une valeur dans la plage de 0 à 20 ;
l'indice r vaut 0 ou est positif et a une valeur dans la plage de 0 à 30 ;
l'indice t vaut 0 ou 1 ;
l'indice u vaut 0 ou 1 ;
l'indice v vaut 0 ou est positif et a une valeur dans la plage de 0 à 100 sous réserve que $(v+w+x) > 0$ ;
l'indice w vaut 0 ou est positif et a une valeur dans la plage de 0 à 100 sous réserve que $(v+w+x) > 0$ ;
l'indice x vaut 0 ou est positif et a une valeur dans la plage de 0 à 100 sous réserve que $(v+w+x) > 0$ ; et

a2) un aminosilane de formule :

$$N(H)(R^1)R^2Si(OR^3)_{3-a-b-c}(OR^4)_a(R^5Si(OR^6)_d(R^7)_e)_bR^8_c,$$

avec

$R^1$ est choisi dans le groupe constitué par H ou un radical hydrocarboné monovalent contenant 1 à 20 atomes de carbone ;
$R^2$ est choisi dans un groupe constitué par un radical hydrocarboné linéaire ou ramifié divalent constitué

de 1 à 60 atomes de carbone ;
$R^4$ est un radical hydrocarboné qui contient 3 à 200 atomes de carbone ;
$R^5$ est choisi dans un groupe constitué de l'oxygène ou d'un radical hydrocarboné linéaire ou ramifié divalent constitué de 1 à 60 atomes de carbone ;
$R^3$, $R^6$, $R^7$ et $R^8$ et sont chacun indépendamment choisis dans le groupe de radicaux hydrocarbonés linéaires ou ramifiés monovalents ayant 1 à 200 atomes de carbone ;
l'indice b vaut 0 ou est un nombre positif et a une valeur dans la plage de 0 à 3 ;
l'indice a est un nombre positif inférieur à 3 ;
les indices b et c valent 0 ou sont positifs et ont une valeur dans la plage de 0 à 3 sous réserve que $(a+b+c) \leq 3$ ;
les indices d et e valent 0 ou sont positifs et ont une valeur dans la plage de 0 à 3 sous réserve que $(d+e) = 3$ ; et

(b) un agent de coloration capillaire.

**2.** Composition de coloration capillaire selon la revendication 1, dans laquelle le polymère d'aminosilicone filmogène est le produit de la réaction du siloxane (a1), de l'aminosilane (a2) et d'un composé (I) de formule :

$$\text{(I)} \qquad R^{29}(R^{30})_{\kappa}Si(OR^{31})_{3-\eta-\theta}(R^{32})_{\eta}(OR^{33})_{\theta},$$

où

$R^{29}$ est un radical hydrocarboné monovalent contenant une ou plusieurs fractions d'oxirane ou d'oxétane ayant de 3 à 12 atomes de carbone ;
$R^{30}$ est un radical hydrocarboné divalent constitué par 1 à 60 atomes de carbone et l'indice $\kappa$ a une valeur de 0 ou 1 ;
$R^{31}$ et $R^{32}$ sont indépendamment choisis dans le groupe de radicaux hydrocarbonés linéaires ou ramifiés monovalents ayant de 1 à 60 atomes de carbone ;
l'indice $\eta$ vaut zéro ou est positif et a une valeur dans la plage de 0 à 3 ;
l'indice $\theta$ est $> 0$ et $\leq 3$, sous réserve que $3-\eta-\theta \geq 0$ ; $R^{33}$ est un radical hydrocarboné qui contient 3 à 200 atomes de carbone.

**3.** Composition de coloration capillaire selon la revendication 2, dans laquelle :

$R^1$ a de 1 à 5 atomes de carbone ;
$R^2$ a de 2 à 8 atomes de carbone ;
$R^4$ a de 3 à 8 atomes de carbone ;
$R^3$, $R^6$, $R^7$ et $R^8$ ont chacun indépendamment de 1 à 15 atomes de carbone ;
l'indice a est dans la plage de 2 à 3 ;
l'indice b est dans la plage de 0 à 15 ;
l'indice c est dans la plage de 0 à 2 ;
$R^9$, $R^{10}$, $R^{11}$, $R^{12}$, $R^{13}$, $R^{18}$, $R^{19}$, $R^{20}$ et $R^{21}$ ont chacun indépendamment de 1 à 3 atomes de carbone ;
l'indice k est dans la plage de 5 à 250 ;
les indices v, w et x sont chacun indépendamment dans la plage de 0 à 35 ;
$R^{31}$ et $R^{32}$ ont chacun indépendamment de 1 à 8 atomes de carbone, et
$R^{33}$ a de 3 à 50 atomes de carbone.

**4.** Composition de coloration capillaire selon la revendication 2, dans laquelle :

$R^1$ est H ;
$R^2$ a de 2 à 5 atomes de carbone ;
$R^4$ a de 3 à 5 atomes de carbone ;
$R^3$, $R^6$, $R^7$ et $R^8$ ont chacun indépendamment de 2 à 8 atomes de carbone ;
l'indice a est dans la plage de 0 ou 1 ;
l'indice b vaut 3 ;
l'indice c vaut 0 ou 1 ;
$R^9$, $R^{10}$, $R^{11}$, $R^{12}$, $R^{13}$, $R^{18}$, $R^{19}$, $R^{20}$ et $R^{21}$ sont chacun indépendamment un groupe méthyle ;
l'indice k est dans la plage de 5 à 150 ;

les indices v, w et x sont chacun indépendamment dans la plage de 0 à 25 ;
$R^{31}$ et $R^{32}$ ont chacun indépendamment de 1 à 4 atomes de carbone ; et
$R^{33}$ a de 3 à 10 atomes de carbone.

**5.** Composition de coloration capillaire selon la revendication 1, dans laquelle :

$R^1$ est H ;
$R^2$ a de 2 à 5 atomes de carbone ;
$R^3$ a de 3 à 5 atomes de carbone ;
$R^4$ a de 3 à 5 atomes de carbone ;
$R^3$, $R^6$, $R^7$ et $R^8$ ont chacun indépendamment de 2 à 8 atomes de carbone ;
l'indice a vaut 1 ;
l'indice b vaut 0 ;
l'indice c vaut 0 ;
$R^9$, $R^{10}$, $R^{11}$, $R^{12}$, $R^{13}$, $R^{18}$, $R^{19}$, $R^{20}$ et $R^{21}$ sont chacun indépendamment un groupe méthyle ;
l'indice k est dans la plage de 5 à 150 ;
les indices v, w et x sont chacun indépendamment dans la plage de 0 à 25 ;
l'indice n vaut 0.

**6.** Composition de coloration capillaire selon la revendication 1, dans laquelle :

le siloxane est un polysiloxane à encapsulation époxy de structure moyenne :

$$CH_2(O)CHCH_2O(CH_2)_\lambda Si(CH_3)_2O[Si(CH_3)_2O]_kSi(CH_3)_2CH_2CH_2CH_2OCH_2C\ H(O)CH_2$$

avec l'indice k est dans la plage de 5 à 250, de 5 à 150, de préférence de 50 à 100, et
l'indice $\lambda$ vaut 2 ou 3 ; et

l'aminosilane est de formule $H_2NR^2Si(OR^3)_2(OR^4)$, avec $R^2$ a de 2 à 5 atomes de carbone ;
$R^3$ a de 3 à 5 atomes de carbone ; et
$R^4$ a de 3 à 5 atomes de carbone.

**7.** Composition de coloration capillaire selon la revendication 6, dans laquelle le polymère d'aminosilicone filmogène est le produit de la réaction du siloxane (a1), de l'aminosilane (a2) et d'un polyéther encapsulé de structure moyenne $CH_2(O)CHCH_2O(CH_2CH_2O)_\mu CH_2CH(O)CH_2$, où l'indice $\beta$ est dans la plage de 10 à 20, ou un polyιther encapsul ι de structure moyenne $CH_2(O)CHCH_2O(CH_2CH_2O)_\mu(CH_2CHCH_3O)_\nu CH_2CH(O)CH_2$, où l'indice $\mu$ est dans la plage de 1 à 20 et l'indice $\nu$ est dans la plage de 1 à 20.

**8.** Composition de coloration capillaire selon la revendication 7, dans laquelle le polymère d'aminosilicone filmogène est le produit de la réaction du siloxane (a1), de l'aminosilane (a2) en présence d'une amine primaire ou secondaire, de préférence une amine primaire choisie dans le groupe constitué par la méthylamine, l'éthylamine, la propylamine, l'éthanolamine, l'isopropylamine, la butylamine, l'isobutylamine, l'hexylamine, la dodécylamine, l'oléylamine, l'aniline aminopropyltriméthylsilane, l'aminopropyltriéthylsilane, l'aminomorpholine, la 3-(diéthylamino)propylamine, la benzylamine, le naphtylamine 3-amino-9-éthylcarbazole, le 1-aminoheptaphlorohexane, la 2,2,3,3,4,4,5,5,6,6,7,7,8,8,8-pentadécafluoro-1-octanamine et des mélanges de ceux-ci ou une amine secondaire choisie dans le groupe constitué par la méthyléthylamine, la méthylhexylamine, la méthyloctadécylamine, la diéthanolamine, la dibenzylamine, la dihexylamine, la dicyclohexylamine, la pipéridine, la pyrrolidine, le phtalimide et des mélanges de ceux-ci.

**9.** Composition de coloration capillaire selon l'une quelconque des revendications précédentes, dans laquelle l'agent de coloration capillaire comprend un ou plusieurs pigments, dans laquelle les un ou plusieurs pigments ont de préférence un diamètre de particule $D_{50}$ de 5 microns à 60 microns.

**10.** Composition de coloration capillaire selon l'une quelconque des revendications précédentes, dans laquelle l'agent de coloration capillaire comprend une ou plusieurs matières colorées, dans laquelle les une ou plusieurs matières colorées sont de préférence choisies dans le groupe constitué par : des fibres colorées, des billes colorées, des particules colorées telles que des nanoparticules, des polymères colorés comprenant des teintures à liaison covalente, des particules ayant des propriétés de diffraction, et des mélanges de ceux-ci.

**11.** Composition de coloration capillaire selon l'une quelconque des revendications 1 à 8, dans laquelle l'agent de coloration capillaire comprend une composition de teinture et éventuellement une composition de révélateur, dans laquelle la composition de révélateur comprend un ou plusieurs agents oxydants ; et dans laquelle la composition de teinture comprend un ou plusieurs précurseurs de teinture oxydants comprenant un ou plusieurs coupleurs et un ou plusieurs intermédiaires primaires ou dans laquelle la composition de teinture est essentiellement constituée d'une ou plusieurs teintures directes.

**12.** Composition de coloration capillaire selon la revendication 11, dans laquelle les un ou plusieurs intermédiaires primaires sont choisis dans le groupe constitué par la toluène-2,5-diamine, la p-phénylènediamine, la N-phényl-p-phénylènediamine, la N,N-bis(2-hydroxyéthyl)-p-phénylènediamine, la 2-hydroxyéthyl-p-phénylènediamine, la hydroxypropyl-bis-(N-hydroxyéthyl-p-phénylènediamine), la 2-méthoxyméthyl-p-phénylènediamine, la 2-(1,2-dihydroxyéthyl)-p-phénylènediamine, le 2,2'-(2-(4-aminophénylamino)éthylazanediyl)diéthanol, le 2-(2,5-diamino-4-méthoxyphényl)propane-1,3-diol, le 2-(7-amino-2H-benzo[b][1,4]oxazin-4(3H)-yl)éthanol, la 2-chloro-p-phénylènediamine, le p-aminophénol, le p-(méthylamino)phénol, le 4-amino-m-crésol, le 6-amino-m-crésol, le 5-éthyl-o-aminophénol, la 2-méthoxy-p-phénylènediamine, le 2,2'-méthylènebis-4-aminophénol, la 2,4,5,6-tétraminopyrimidine, le 2,5,6-triamino-4-pyrimidinol, le sulfate de 1-hydroxyéthyl-4,5-diaminopyrazole, le 4,5-diamino-1-méthylpyrazole, le 4,5-diamino-1-éthylpyrazole, le 4,5-diamino-1-isopropylpyrazole, le 4,5-diamino-1-butylpyrazole, le 4,5-diamino-1-pentylpyrazole, le 4,5-diamino-1-benzylpyrazole, le diméthosulfonate de 2,3-diamino-6,7-dihydropyrazolo[1,2-a]pyrazol-1(5H)-one, le 4,5-diamino-1-hexylpyrazole, le 4,5-diamino-1-héptylpyrazole, le méthoxyméthyl-1,4-diaminobenzène, le N,N-bis(2-hydroxyéthyl)-N-(4-aminophényl)-1,2-diaminoéthane, le chlorhydrate de 2-[(3-aminopyrazolo[1,5-a]pyridin-2-yl)oxy]éthanol, des sels de ceux-ci et des mélanges de ceux-ci.

**13.** Utilisation de la composition de coloration capillaire selon l'une quelconque des revendications 1 à 12 pour raviver des fibres capillaires déjà teintes.

**14.** Procédé permettant de déposer un film sur des fibres de kératine, le procédé comprenant le fait d'appliquer la composition de coloration capillaire selon l'une quelconque des revendications 1 à 12 sur des fibres de kératine et de laisser sécher les fibres de kératine ou de les sécher.

**15.** Trousse comprenant :

◦ la composition de coloration capillaire selon l'une quelconque des revendications 1 à 12 ; et
◦ un applicateur.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2214633 B1
- EP 1084696 A1
- CA 2576189 A1
- EP 2801281 A1


### Non-patent literature cited in the description


- **BARBER et al.** *Pharmaceutical Development and Technology,* 1998, vol. 3 (2), 153-161
- **SAGARIN.** Cosmetic Science and Technology. *Interscience, Special Edn.,* vol. 2, 308-310
- International Cosmetics Ingredient Dictionary and Handbook. The Cosmetics, Toiletry, and Fragrance Association, vol. 2